# EUROPEAN PATENT SPECIFICATION

(11) **EP 1 202 963 B1**
(45) Date of publication and mention of the grant of the patent: **03.01.2007**
(21) Application number: 00944673.3
(22) Date of filing: 11.07.2000
(51) Int. Cl.: C07D 209/22, A61K 31/40

(54) **SPLA2 INHIBITORS**
SPLA2 INHIBITOREN
INHIBITEURS DE SPLA2

(30) Priority: 19.07.1999 US 144502 P
(43) Date of publication of application: 08.05.2002
(73) Proprietor: ELI LILLY AND COMPANY, Indianapolis, Indiana 46285 (US)
(72) Inventor: LIN, Ho-Shen, Indianapolis, IN 46217 (US); RICHETT, Michael, Enrico, Indianapolis, IN 46250 (US)
(74) Representative: Williams, Richard Andrew Norman
(86) International application number: PCT/US2000/016319
(87) International publication number: WO 2001/005761

(56) References cited:
- EP-A- 0 620 215
- EP-A- 0 675 110
- EP-A- 0 952 149
- WO-A-00/37358
- WO-A-91/06537
- WO-A-96/37469
- WO-A-99/21546
- WO-A-99/21559
- US-A- 5 684 034
- DILLARD R D ET AL: "INDOLE INHIBITORS OF HUMAN NONPANCREATIC SECRETORY PHOSPHOLIPASE A21. INDOLE-3-ACETAMIDES" JOURNAL OF MEDICINAL CHEMISTRY,US,AMERICAN CHEMICAL SOCIETY. WASHINGTON, vol. 39, no. 26, 20 December 1996 (1996-12-20), pages 5119-5136, XP002046054 ISSN: 0022-2623

## Description

### Field of the Invention

This invention relates to novel indole compounds useful for Inflammatory Diseases.

### Background of the Invention

The structure and physical properties of human non-pancreatic secretory phospholipase A₂ (hereinafter called, "sPLA₂") has been thoroughly described in two articles, namely, "Cloning and Recombinant Expression of Phospholipase A₂ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K.; The Journal of Biological Chemistry, Vol. 264, No. 10, Issue of April 5, pp. 5335-5338, 1989; and "Structure and Properties of a Human Non-pancreatic Phospholipase A₂" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989; the disclosures of which are incorporated herein by reference.

It is believed that sPLA₂ is a rate limiting enzyme in the arachidonic acid cascade which hydrolyzes membrane phospholipids. Thus, it is important to develop compounds which inhibit sPLA₂ mediated release of fatty acids (e.g., arachidonic acid). Such compounds would be of value in general treatment of conditions induced and/or maintained by overproduction of sPLA_{2;} such as sepsis or rheumatoid arthritis.

It is desirable to develop new compounds and treatments for sPLA₂ induced diseases.

EP 675110 relates to 1H-indole-3-glyoxylamides, EP 620215 to 1H-indole-3-acetamides, US 5684034 to 1-indole-3-acetamides, WO 00/37358 to indole oxime amides, WO 99/21559 to morpholino-N-ethyl ester prodrugs of indoles, WO 99/21546 to N,N-diethylglycolamido ester prodrugs of indoles and EP 952149 relates to substituted carbazoles. All of these compounds are referred to therein as inhibitors of sPLA2 mediated release of fatty acids for treatment of conditions such as septic shock. WO 96/37469 relates to N-benzylindol-3-yl propanoic acid derivatives as cyclooxygenase-2 inhibitors. WO 91/06537 relates to substituted indole-, indene-, pyranoindole- and tetrahydrocarbazole- alkanoic acid derivatives as inhibitors of PLA2 and lipoxygenase J.Med.Chem., 39(1996), pp.5119-5139, describe the syntheses of a series of indole-3-acetamides and related compounds and their structure-activity relationships, and pharmacological activities as hnps-PLA2 inhibitors.

### Summary of the Invention

This invention provides novel indole compounds having potent and selective effectiveness as inhibitors of mammalian sPLA₂.

This invention is also the use of novel indole compounds useful in the treatment and prevention of Inflammatory Diseases.

This invention is also the use of novel of indole compounds to inhibit mammalian sPLA₂ mediated release of fatty acids.

This invention is also a pharmaceutical composition containing any of the indole compounds of the invention.

### I. Definitions:

The term, "Inflammatory Diseases" refers to diseases such as inflammatory bowel disease, sepsis, septic shock, adult respiratory distress syndrome, pancreatitis, trauma-induced shock, bronchial asthma, allergic rhinitis, rheumatoid arthritis, cystic fibrosis, stroke, acute bronchitis, chronic bronchitis, acute bronchiolitis, chronic bronchiolitis, osteoarthritis, gout, spondylarthropathris, ankylosing spondylitis, Reiter's syndrome, psoriatic arthropathy, enterapathric spondylitis, Juvenile arthropathy or juvenile ankylosing spondylitis, Reactive arthropathy, infectious or post-infectious arthritis, gonoccocal arthritis, tuberculous arthritis, viral arthritis, fungal arthritis, syphilitic arthritis, Lyme disease, arthritis associated with "vasculitic syndromes", polyarteritis nodosa, hypersensitivity vasculitis, Luegenec's granulomatosis, polymyalgin rheumatica, joint cell arteritis, calcium crystal deposition arthropathris, pseudo gout, non-articular rheumatism, bursitis, tenosynomitis, epicondylitis (tennis elbow), carpal tunnel syndrome, repetitive use injury (typing), miscellaneous forms of arthritis, neuropathic joint disease (charco and joint), hemarthrosis (hemarthrosic), Henoch-Schonlein Purpura, hypertrophic osteoarthropathy, multicentric reticulohistiocytosis, arthritis associated with certain diseases, surcoilosis, hemochromatosis, sickle cell disease and other hemoglobinopathries, hyperlipoproteineimia, hypogammaglobulinemia, hyperparathyroidism, acromegaly, familial Mediterranean fever, Behat's Disease, systemic lupus erythrematosis, or relapsing polychondritis and related diseases which comprises administering to a mammal in need of such treatment a therapeutically effective amount of the compound of formula I in an amount sufficient to inhibit sPLA₂ mediated release of fatty acid and to thereby inhibit or prevent the arachidonic acid cascade and its deleterious products.

The term, "indole nucleus" refers to a nucleus (having numbered positions) with the structural formula (X) :

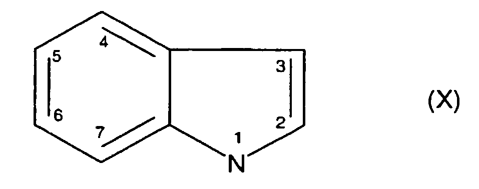

The indole compounds of the invention employ certain defining terms as follows:

The term, "alkyl" by itself or as part of another substituent means, unless otherwise defined, a straight or branched chain monovalent hydrocarbon radical such as methyl, ethyl, n-propyl, isopropyl, n-butyl, tertiary butyl, sec-butyl, n-pentyl, and n-hexyl.

The term, "alkenyl" employed alone or in combination with other terms means a straight chain or branched monovalent hydrocarbon group having the stated number range of carbon atoms, and typified by groups such as vinyl, propenyl, crotonyl, isopentenyl, and various butenyl isomers.

The term, "hydrocarbyl" means an organic group containing only carbon and hydrogen.

The term, "halo" means fluoro, chloro, bromo, or iodo.

The terms, "amino acid residue" refer to the portion of the amino acid group coupled at the nitrogen atom of the amino terminus. It is the amino acid less a hydrogen atom from the amino terminus. It is further illustrated as used herein for the amino acid alanine attached at the nitrogen atom as shown below:

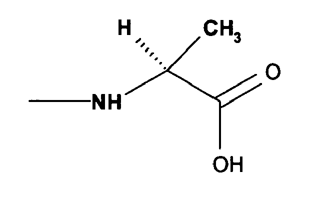

The term, "amine", includes primary, secondary and tertiary amines.

The terms, "mammal" and "mammalian" include human and domesticated quadrupeds.

The term, "alkylene chain of 1 or 2 carbon atoms" refers to the divalent radicals, -CH₂-CH₂- and -CH₂-.

The term "oxime amide" means the radical, -C=NOR-C(O)NH₂.

### II. The amino acid 1H-indole Compounds of the Invention:

The present invention provides novel classes of indole compounds useful as sPLA2 inhibitors for the treatment of inflammation. Classes of indole compounds of this invention include indole glyoxylamide amino acid derivatives, indole-3-oxime amide amino acid derivatives and indole acetamide amino acid derivatives. The compounds of the invention have the general formula (I), or a pharmaceutically acceptable salt, solvate, or prodrug derivative selected from the methyl, ethyl, propyl, isopropyl, n-butyl, isobutyl, tert-butyl, morpholinoethyl, and N,N-diethylglycolamido esters thereof; 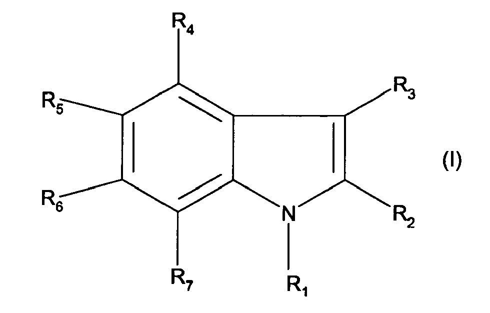 wherein ;
R₁ is the group -(L₁)-R₁₁; where, -(L₁)- is selected from a group represented by the formulae (Ia), (Ib), (Ic), (Id), (Ie), and (If):

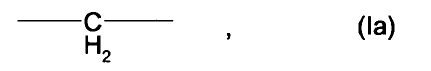

   -O- , (Ib)

   -S- , (Ic)

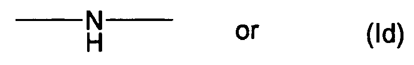

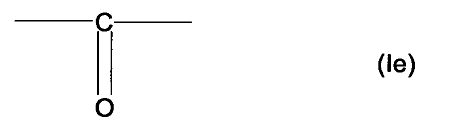

   or 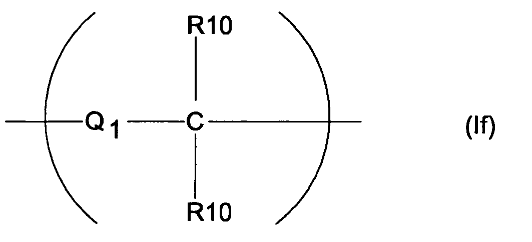 where Q₁ is a bond or any of the divalent groups Ia, Ib, Ic, Id, and Ie and R₁₀ is independently -H, C₁₋₈ alkyl, C₁₋₈ haloalkyl or C₁₋₈ alkoxy and where R₁₁ is a substituted or unsubstituted carbocyclic radical selected from the group consisting of C₅-C₁₄ cycloalkyl, C₅-C₁₄ cycloalkenyl, phenyl, spiro[5.5]undecanyl, naphthyl, norbornanyl, bicycloheptadienyl, tolulyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (a): 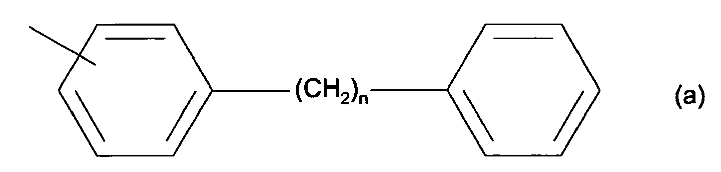 where n is a number from 1 to 8;
R₂ is hydrogen, C₁-C₄ alkyl, C₂-C₄ alkenyl, -O-(C₁-C₃ alkyl), -S-(C₁-C₃ alkyl), C₃-C₄ cycloalkyl, -CF₃, halo, -NO₂, -CN, or -SO₃;
R₃ is -(L₃)- Z, where -(L₃)- is a bond and Z is selected from a group represented by the formulae, 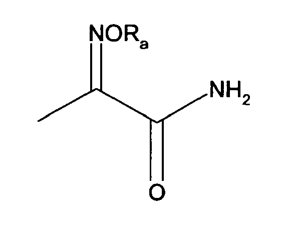 wherein Rₐ is hydrogen, methyl, ethyl, propyl, isopropyl, phenyl or benzyl, 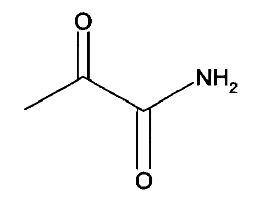 or 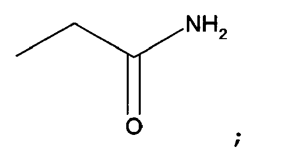
R₄ is the group, -(L_{c})-(acylamino acid group); wherein -(L_{c})- is a divalent group selected from,

   ⁅-O-CH₂-⁆,

   ⁅-S-CH₂-⁆,

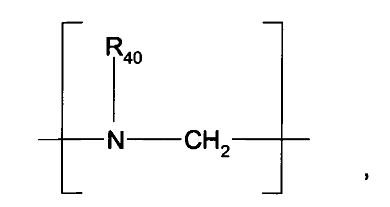

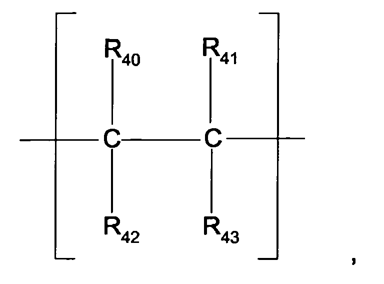

   or 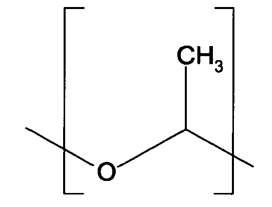 where R₄₀, R₄₁, R₄₂, and R₄₃ are each independently selected from hydrogen, C₁-C₈ alkyl and wherein the (acylamino acid group) is: 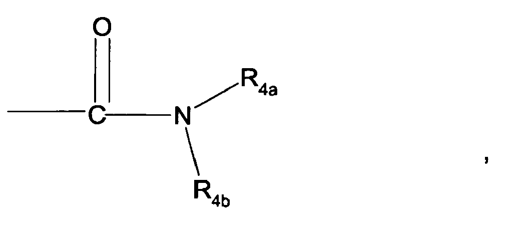 and R^{4a} is selected from the group consisting of H, (C₁-C₆)alkyl, (C₁-C₆)alkoxy and aryl; and wherein NR^{4b} is an amino acid residue selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine *l-*proline, and *d*-proline;
   R₅ is hydrogen; and
   R₆ and R₇ are hydrogen;
   provided that the compound 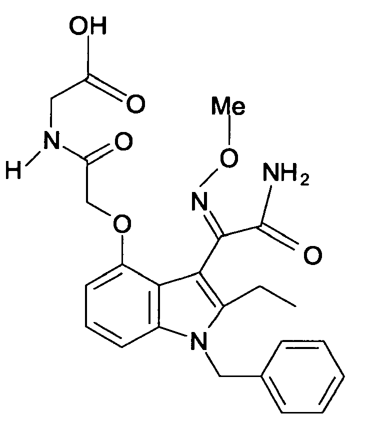 is excluded.

### Preferred Subgroups of Compounds of Formula (I):

### Preferred R₁ substituents:

A preferred subclass of compounds of formula (I) are those where the linking group -(L₁)- of R₁ is an alkylene chain of 1 or 2 carbon atoms, namely, -(CH₂)- or -(CH₂-CH₂)-.

Particularly preferred are compounds wherein for R₁ the combined group -(L₁)-R₁₁ is selected from the groups; 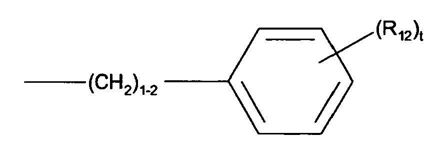 or 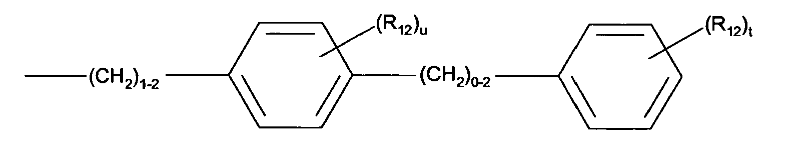 where R₁₂ is a radical independently selected from halo, C₁-C₁₀ alkyl, C₁-C₁₀ alkoxy, -S-(C₁-C₁₀ alkyl), C₁-C₁₀ haloalkyl, and C₁-C₁₀ hydroxyalkyl and t is a number from 0 to 5 and u is a number from 0 to 4.

### Preferred R₂ substituents:

R₂ is preferably selected from the group consisting of hydrogen, methyl, ethyl, propyl, isopropyl, cyclopropyl, -F, -CF₃, -Cl, -Br, or -O-CH₃.

### Preferred R₃ substituents:

A preferred subclass of compounds of formula (I) are those wherein Z is an oxime amide group.

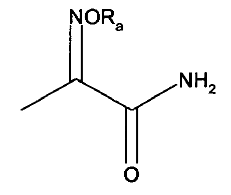

Also preferred are compounds of formula (I) wherein R₃ is an oxime amide group and Rₐ is hydrogen, methyl or ethyl.

### Preferred R₄ substituents:

Another preferred subclass of compounds of formula (I) are those wherein R₄ is a substituent having an acylamino acid linker -(L_{c})-, selected from the specific groups;

⁅O-CH₂⁆ ,

⁅S-CH₂⁆ ,

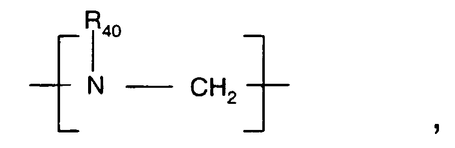

⁅CH₂-CH₂⁆,

or 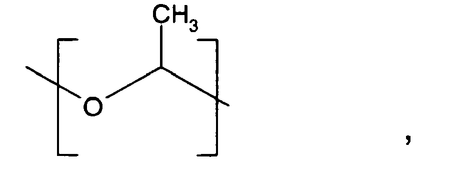 where R₄₀ is hydrogen or C₁ -C₈ alkyl.
Preferred as the (acylamino acid group) in the group R₄ is the group: 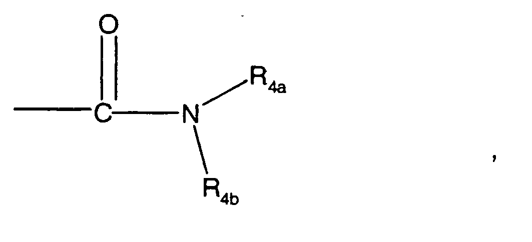 wherein R⁴a is H. A preferred NR^{4b} group is an amino acid residue wherein the amino acid group is selected from the group comprising isoleucine, valine, phenylalanine, aspartic acid, leucine, glycine and isomers and derivatives thereof. A salt or a prodrug derivative of the (acylamino acid group) is also a suitable substituent.

Particularly preferred are R^{4b} groups that combine with the nitrogen atom to represent amino acid residues from the amino acid groups selected from: glycine, glycine methyl ester, L-alaninie, L-alanine methylester, L-leucine, L-leucine methyl ester, L-aspartic acid, L-aspartic acid dimethyl ester, L-phenyl alanine, L-phenylalanine methyl ester, malonic acid, malonic acid dimethylester, L- valine, L-valine methyl ester, L-isoleucine, L-isoleucine methyl ester, or salt, and derivatives thereof.

Most preferred as non-interfering substituents are methyl, ethyl, propyl, and isopropyl.

Preferred compounds of the invention are those having the general formula (II), or a pharmaceutically acceptable salt, solvate, or prodrug selected from the methyl, ethyl, propyl, isopropyl, n-butyl, isobutyl, tert-butyl, morpholinoethyl, and N,N-diethylglycolamido esters thereof; 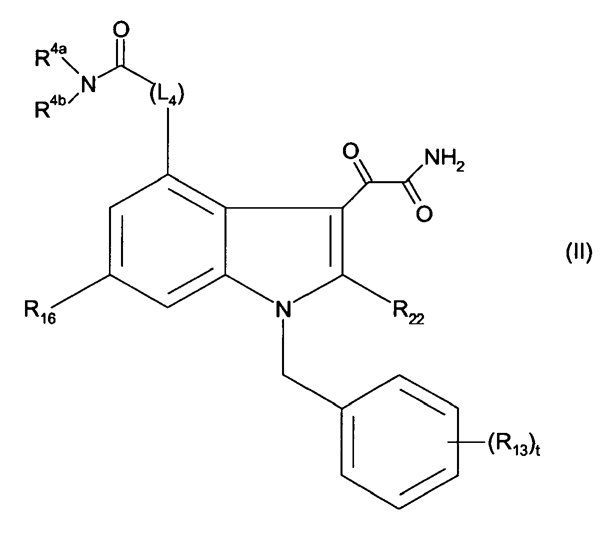 wherein ;
R₂₂ is selected from hydrogen, methyl, ethyl, propyl, isopropyl, cyclopropyl, -F, -CF₃, -Cl, -Br, or -O-CH₃;
R^{4a} is hydrogen; and
NR^{4b} is an amino acid residue selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine, *1*-proline, and *d*-proline, and -(L₄)- is a divalent group selected from;

   ⁅O-CH₂⁆,

   ⁅S-CH₂⁆,

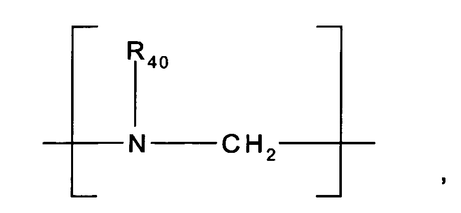

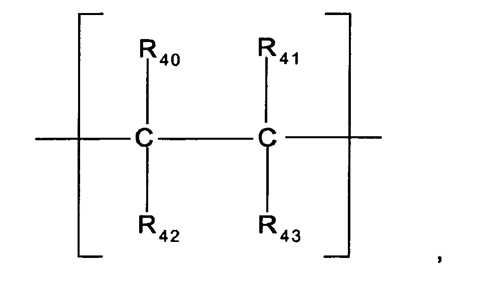

   or 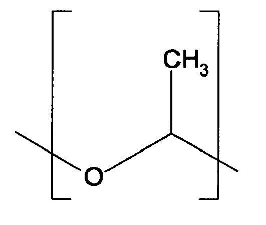 where R₄₀, R₄₁, R₄₂, and R₄₃ are each independently selected from hydrogen or C₁-C₈ alkyl;
   R₁₆ is hydrogen; and
   R₁₃ is selected from hydrogen and C₁-C₈ alkyl, C₁-C₈ alkoxy, -S-(C₁-C₈ alkyl), C₁-C₈ haloalkyl, C₁-C₈ hydroxyalkyl, phenyl, halophenyl, and halo, and t is an integer from 0 to 5.

Preferred specific compounds (and all pharmaceutically acceptable salts, solvates and prodrug derivatives selected from the methyl, ethyl, propyl, isopropyl, n-butyl, isobutyl, tert-butyl, morpholinoethyl, and N,N-diethylglycolamido esters thereof) which are illustrative of the compounds of the invention are as follow:
*N*-[2-[[3-(Aminooxoacetyl)-2-ethyl-1-(phenylmethyl)-1H-indol-4-yl]oxy]acetyl]glycine ;
*N*-[2-[[3-(Aminooxoacetyl)-2-ethyl-1-(phenylmethyl)-1H-indol-4-yl]oxy]acetyl]glycine methyl ester;
*N*-[2-[[3-(Aminooxoacetyl)-2-ethyl-1-(phenylmethyl)-1H-indol-4-yl]oxy]acetyl]-L-alanine;
*N*-[2-[[3-(Aminooxoacetyl)-2-ethyl-1-(phenylmethyl)-1H-indol-4-yl]oxy]acetyl]-L-alanine methyl ester;
*N*-[2-[[3-(Aminooxoacetyl)-2-ethyl-1-(phenylmethyl)-1H-indol-4-yl]oxy]acetyl]-L-leucine;
*N*-[2-[[3-(Aminooxoacetyl)-2-ethyl-1-(phenylmethyl)-1H-indol-4-yl]oxy]acetyl]-L-leucine methyl ester;
*N*-[2-[[3-(Aminooxoacetyl)-2-ethyl-1-(phenylmethyl)-1H-indol-4-yl]oxy]acetyl]-L-aspartic acid;
*N*-[2-[[3-(Aminooxoacetyl)-2-ethyl-1-(phenylmethyl)-1H-indol-4-yl]oxy]acetyl]-L-aspartic acid dimethyl ester;
*N*-[2-[[3-(Aminooxoacetyl)-2-ethyl-1-(phenylmethyl)-1H-indol-4-yl]oxy]acetyl]-L-phenylalanine;
*N*-[2-[[3-(Aminooxoacetyl)-2-ethyl-1-(phenylmethyl)-1H-indol-4-yl]oxy]acetyl]-L-phenylalanine methyl ester;
[2-[[3-(Aminooxoacetyl)-2-ethyl-1-(phenylmethyl)-1H-indol-4-yl]oxy]acetamido]malonic acid;
[2-[[3-(Aminooxoacetyl)-2-ethyl-1-(phenylmethyl)-1H-indol-4-yl]oxy]acetamido]malonic acid dimethyl ester;
*N*-[2-[[3-(Aminooxoacetyl)-2-ethyl-1-(phenylmethyl)-1H-indol-4-yl]oxy]acetyl]-L-valine;
*N*-[2-[[3-(Aminooxoacetyl)-2-ethyl-1-(phenylmethyl)-1H-indol-4-yl]oxy]acetyl]-L-valine methyl ester;
*N*-[2-[[3-(Aminooxoacetyl)-2-ethyl-1-(phenylmethyl)-1H-indol-4-yl]oxy]acetyl]-L-isoleucine; and
*N*-[2-[[3-(Aminooxoacetyl)-2-ethyl-1-(phenylmethyl)-1H-indol-4-yl]oxy]acetyl]-L-isoleucine methyl ester.

The salts of the above indole compounds represented by formulae (I) and (II) are an additional aspect of the invention. In those instances where the compounds of the invention possess acidic or basic functional groups various salts may be formed which are more water soluble and physiologically suitable than the parent compound. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, *et al.*, "Pharmaceutical Salts," J. Phar. Sci., 66: 1-19 (1977)). Moreover, the basic group(s) of the compound of the invention may be reacted with suitable organic or inorganic acids to form salts such as acetate, benzenesulfonate, benzoate, bicarbonate, bisulfate, bitartrate, borate, bromide, camsylate, carbonate, chloride, clavulanate, citrate, chloride, edetate, edisylate, estolate, esylate, fluoride, fumarate, gluceptate, gluconate, glutamate, glycolylarsanilate, hexylresorcinate, bromide, chloride, hydroxynaphthoate, iodide, isothionate, lactate, lactobionate, laurate, malate, malseate, mandelate, mesylate, methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate.

Certain compounds of the invention may possess one or more chiral centers and may thus exist in optically active forms. Likewise, when the compounds contain an alkenyl or alkenylene group there exists the possibility of cis- and trans- isomeric forms of the compounds. The R- and S-isomers and mixtures thereof, including racemic mixtures as well as mixtures of cis- and trans- isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods. For example, a racemic mixture may be reacted with a single enantiomer of some other compound. This changes the racemic form into a mixture of diastereomers and diastereomers, because they have different melting points, different boiling points, and different solubilities can be separated by conventional means, such as crystallization.

Prodrugs are derivatives of the compounds of the invention which have chemically or metabolically cleavable groups and become by solvolysis or under physiological conditions the compounds of the invention which are pharmaceutically active in vivo. Derivatives of the compounds of this invention have activity in both their acid and base derivative forms, but the acid derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acidic compound with a suitable alcohol, or amides prepared by reaction of the parent acid compound with a suitable amine. Simple aliphatic or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters. Particularly preferred esters as prodrugs are methyl, ethyl, propyl, isopropyl, n-butyl, isobutyl, tert-butyl, morpholinoethyl, and N,N-diethylglycolamido.

N,N-diethylglycolamido ester prodrugs may be prepared by reaction of the sodium salt of a compound of Formula (I) (in a medium such as dimethylformamide) with 2-chloro-N,N-diethylacetamide (available from Aldrich Chemical Co., Milwaukee, Wisconsin USA; Item No. 25,099-6).

Morpholinylethyl ester prodrugs may be prepared by reaction of the sodium salt of a compound of Formula (I) (in a medium such as dimethylformamide) 4-(2-chloroethyl)morpholine hydrochloride (available from Aldrich Chemical Co., Milwaukee, Wisconsin USA, Item No. C4,220-3).

One of skill in the art is aware that the derivatives of the acid such as the acid salt or the methyl ester of the acid, can be reacted with the amino acid or derivatives thereof to obtain the protected compound 2 or a corresponding derivative. Such methods are well known in the arts and can be found in reference texts such as for example J. March Advanced Organic Chemistry, Wiley Interscience publishers, New York, N.Y, 1985, and R. C. Larock Comprehensive Organic Transformations, VCH Publishers, New York, N.Y, 1989. The protected compounds of formula (2) are also useful sPLA₂ inhibitors and are also compounds of this invention.
The indole-3-oxime compounds of the invention can be prepared following protocol of scheme 2 below;

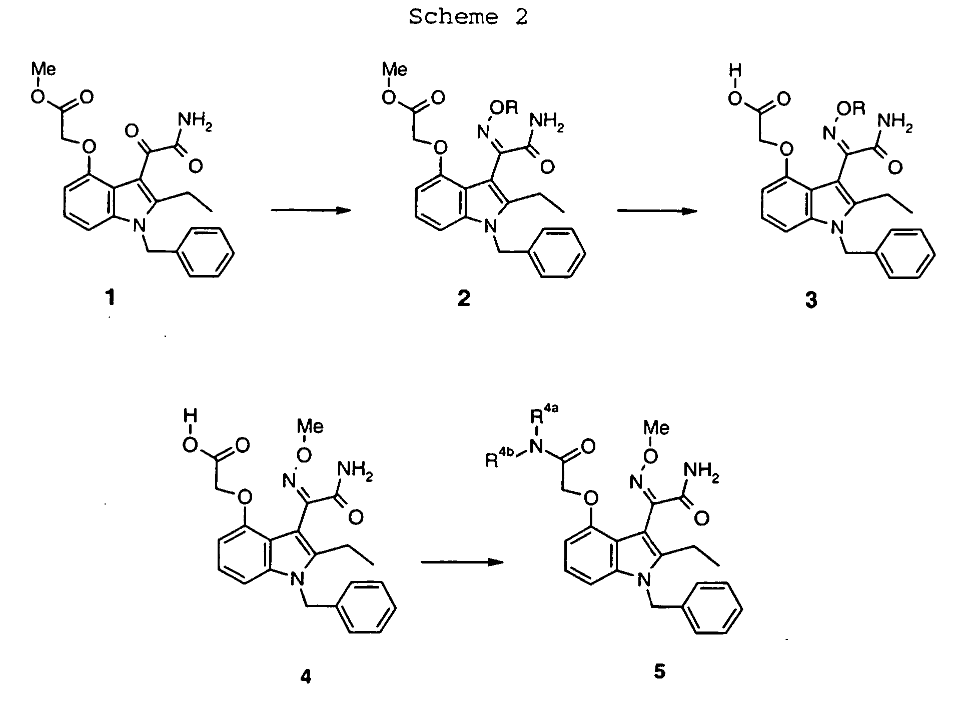

To introduce the oxime functionality, the methyl ester of the glyoxylamide (compound 10 in scheme 1, compound 1 in scheme 2, *supra.*) is heated with hydroxylamine hydrochloride (when R is H) in a THF/methanol mixture for 8 hours or until the reaction was deemed complete. The reaction product is isolated by chromatography or other known laboratory procedure to afford a white solid. Substituted oximes such as when R is methyl, ethyl, phenyl or other substituent can be prepared by reacting the corresponding substituted hydroxylamine hydrochloride or free base with the glyoxylamide as described *supra*. The ester functionality at the 4 or 5 position on the indole nucleus, as in for example, compound 2, can be: (a) converted to the acid by hydrolysis using lithium hydroxide or other known ester hydrolysis methods to afford compounds of formula 3, or (b) converted to an amide functionality directly or via the acid functionality to afford compounds of formula 4. General procedures for the conversion of organic acids to amino acid are well known to artisans in the field, and have been documented in general reference texts including, for example, J. March Advanced Organic Chemistry, Wiley Interscience publishers, New York, N.Y, 1985, and R. C. Larock Comprehensive Organic Transformations, VCH Publishers, New York, N.Y, 1989.

The oxime acid compounds of formula 3 such as the methyloxime compound such as that of formula 4 can be converted to the corresponding amino acid derivative via the methylester by coupling with various amino acids by general coupling procedures known to one skilled in the art. Additional references, or procedures are found in J. March Advanced Organic Chemistry, Wiley Interscience publishers, New York, N.Y, 1985; R. C. Larock Comprehensive Organic Transformations, VCH Publishers, New York, N.Y, 1989 and J. Jones Amino Acids and Peptide Synthesis, Oxford Science Publications, Stephen G. Davis, Editor, Oxford University Press Inc., New York, NY, 1992.

### III. Method of Making the 1H-Indole-3-Glyoxylamide Starting Material for Preparing the Compounds of the Invention:

The synthesis of the indole compounds of the invention (viz., Compounds of Formulae I and II) can be accomplished by well known methods as recorded in the chemical literature. In particular, the indole starting materials may be prepared by the synthesis schemes taught in US Patent No. 5,654,326; the disclosure of which is incorporated herein by reference. Another method of making 1H-indole-3-glyoxylamide sPLA₂ inhibitors is described in United States Patent Application Serial No. 09/105381, filed June 26, 1998 and titled, "Process for Preparing 4-substituted 1-H-Indole-3-glyoxyamides" the entire disclosure of which is incorporated herein by reference.

United States Patent Application Serial No. 09/105381 discloses the following process having steps (a) thru (i):
Preparing a compound of the formula (Iz) or a pharmaceutically acceptable salt or prodrug derivative thereof 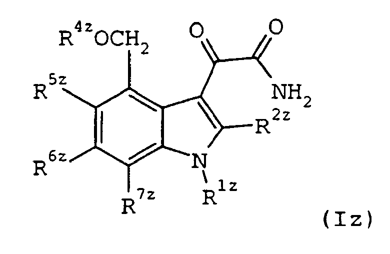 wherein:
R^{1z} is selected from the group consisting of -C₇-C₂₀ alkyl, 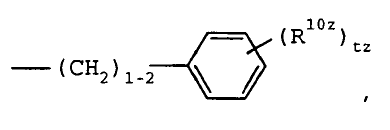 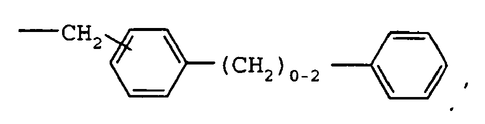 and 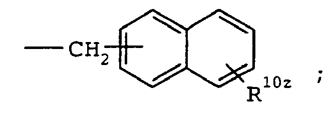 where
R^{10Z} is selected from the group consisting of halo, C₁-C₁₀ alkyl, C₁-C₁₀ alkoxy, -S-(C₁-C₁₀ alkyl) and halo(C₁-C₁₀)alkyl, and tz is an integer from 0 to 5 both inclusive;
R^{2z} is selected from the group consisting of hydrogen, halo, C₁-C₃ alkyl, C₃-C₄ cycloalkyl, C₃-C₄ cycloalkenyl, -O-(C₁-C₂ alkyl), -S-(C₁-C₂ alkyl), aryl, aryloxy and HET;
R^{4z} is the group -CO₂H, or salt and prodrug derivative thereof; and
R^{5z}, R^{6z} and R^{7z} are each independently selected from the group consisting of hydrogen, (C₁-C₆)alkyl, (C₁-C₆)alkoxy, halo(C₁-C₆)alkoxy, halo(C₂-C₆)alkyl, bromo, chloro, fluoro, iodo and aryl;
   which process comprises the steps of:
   a) halogenating a compound of formula Xz 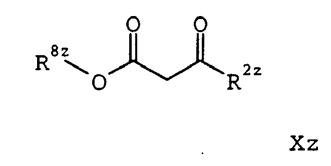 where R^{8Z} is (C₁-C₆)alkyl, aryl or HET; with SO₂Cl₂ to form a compound of formula IX 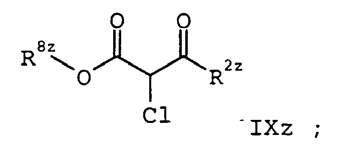
   b) hydrolyzing and decarboxylating a compound of formula IXz 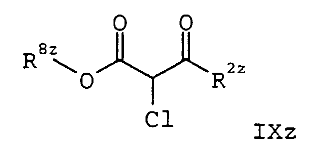 to form a compound of formula VIIIz 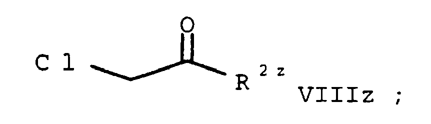
   c) alkylating a compound of formula VIIz 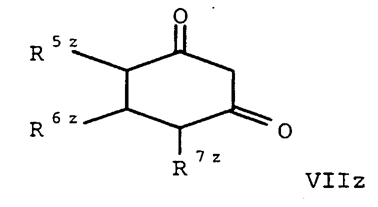 with a compound of formula VIIIz 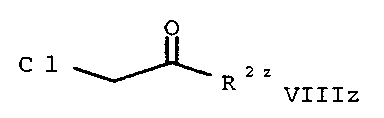 to form a compound of formula VIz 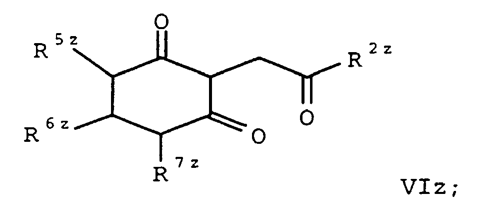
   d) aminating and dehydrating a compound of formula VIz 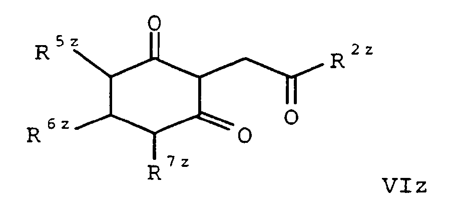 with an amine of the formula R^{1z}NH₂ in the presence of a solvent that forms and azeotrope with water to form a compound of formula Vz;
   e) oxidizing a compound of formula Vz 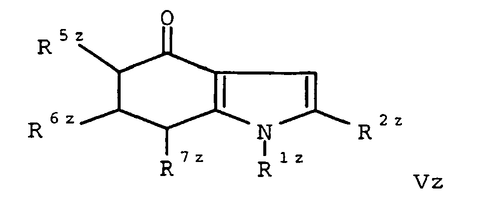 by refluxing in a polar hydrocarbon solvent having a boiling point of at least 150 °C and a dielectric constant of at least 10 in the presence of a catalyst to form a compound of formula IVz 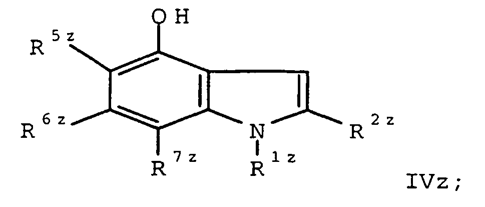
   f) alkylating a compound of the formula IVz 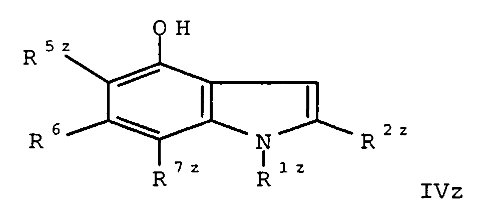 with an alkylating agent of the formula XCH₂R^{4az} where X is a leaving group and R^{4az} is -CO₂R^{4b}, where R^{4bz} is an acid protecting group to form a compound of formula IIIz 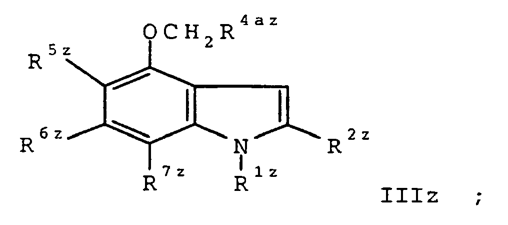
   g) reacting a compound of formula IIIz 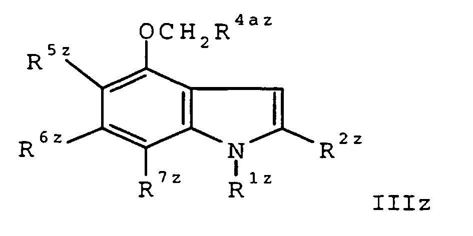 with oxalyl chloride and ammonia to form a compound of formula IIz 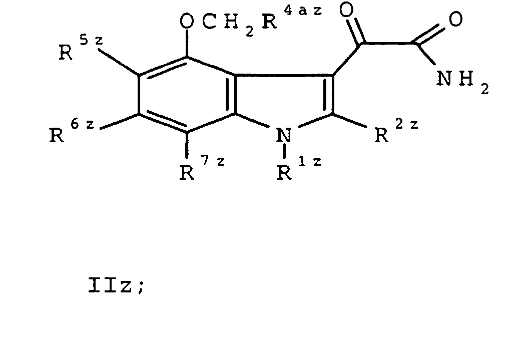 and
   h) optionally hydrolyzing a compound of formula IIz 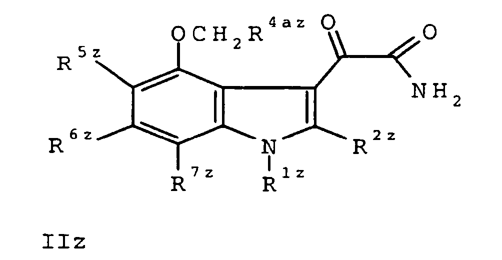 to form a compound of formula Iz.

An alternative protocol useful for the synthesis of the starting material is shown in Scheme 1 below: 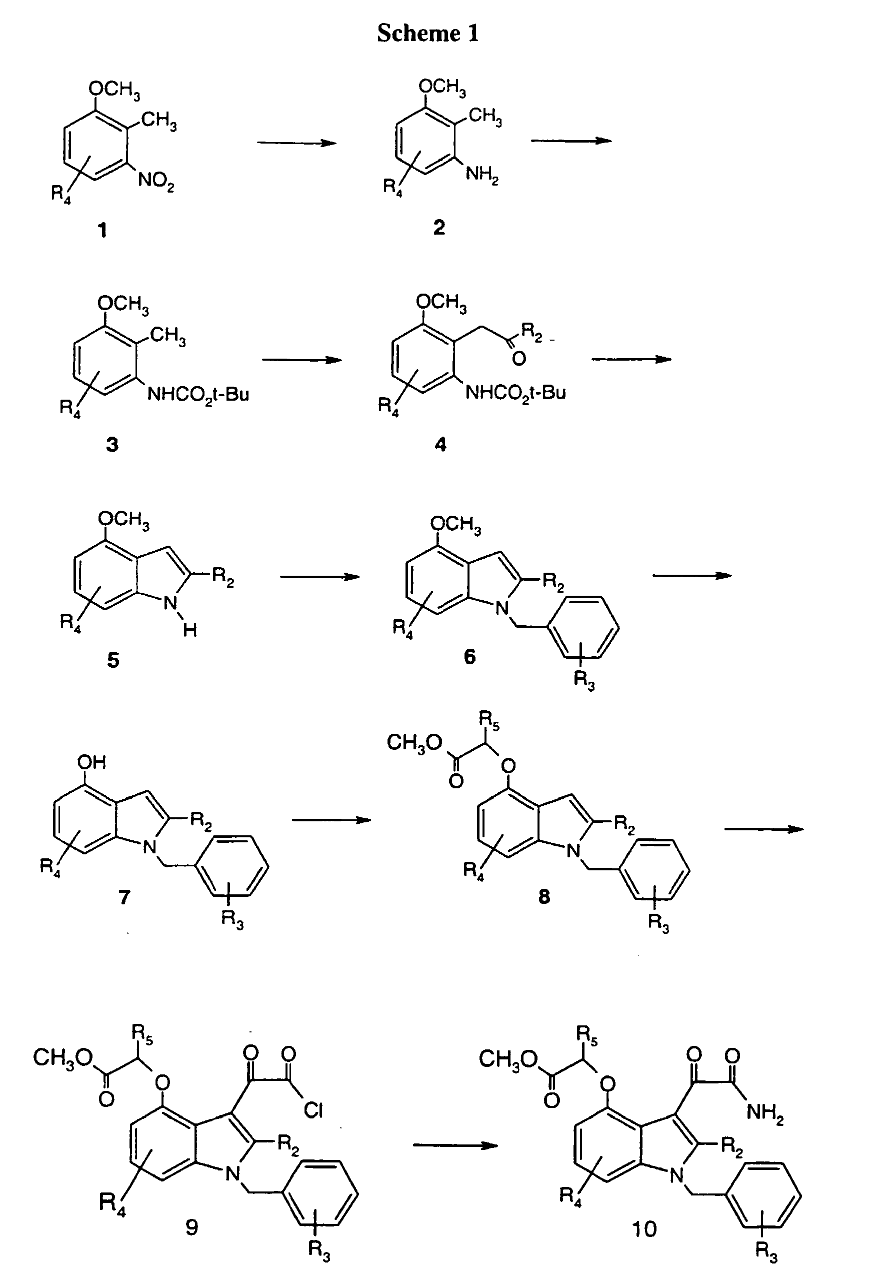 The synthesis of indole-3-oxime amides (compound of formula I and II, supra.) of this invention uses as starting material the glyoxamide ((3-(2-amino-1,2-dioxoethyl)-2-methyl-1-(phenylmethyl)-1H-indol-4-yl)oxy)acetic acid methyl ester, compound 10, *supra.* This starting material is prepared as set out in the preceding section or by the method of Example 9 of U.S. Patent No. 5,654,326 (the disclosure of which is incorporated herein by reference).

To obtain the glyoxylamide starting material substituted in the 4-position with an (acidic group) linked through an oxygen atom, the reactions outlined in the scheme *supra,* are used (for conversions 1 through 5, see ref. Robin D. Clark, Joseph M. Muchowski, Lawrence E. Fisher, Lee A. Flippin, David B. Repke, Michel Souchet, *Synthesis,* 1991, 871-878, the disclosures of which are incorporated herein by reference). The starting material ortho-nitrotoluene, 1, is readily reduced to 2-methyl,3-methoxyaniline, 2. Reduction of 1 is by the catalytic hydrogenation of the corresponding nitrotoluene using palladium on carbon as catalyst. The reduction can be carried out in ethanol or tetrahydrofuran (THF) or a combination of both, using a low pressure of hydrogen. The aniline 2, obtained, is converted to the N-tert-butyloxycarbonyl derivative 3, in good yield, on heating with di-tert-butyl dicarbonate in THF at reflux temperature. The dilithium salt of the dianion of 3 is generated at -40 to -20°C in THF using sec-butyllithium and reacted with the appropriately substituted N-methoxy-N-methylalkanamide to form the ketone 4. This product (4) may be purified by crystallization from hexane, or reacted directly with trifluoroacetic acid in methylene chloride to give the 1,3-unsubstituted indole 5. The 1,3-unsubstituted indole 5 is reacted with sodium hydride in dimethylformamide at room temperature (20-25°C) for 0.5-1.0 hour. The resulting sodium salt of 5 is treated with an equivalent of arylmethyl halide and the mixture stirred at a temperature range of 0-100°C, usually at ambient room temperature, for a period of 4 to 36 hours to give the 1-arylmethylindole, 6. This indole, 6, is O-demethylated by stirring with boron tribromide in methylene chloride for approximately 5 hours (see ref. Tsung-Ying Shem and Charles A Winter, *Adv. Drug Res.,* 1977, 12, 176, the disclosure of which is incorporated herein by reference). The 4-hydroxyindole, 7, is alkylated with an alpha bromoalkanoic acid ester in dimethylformamide (DMF) using sodiumhydride as a base, with reaction condition of 5 to 6. The α-[(indol-4-yl)oxy]alkanoic acid ester, 8, is reacted with oxalyl chloride in methylene chloride to give 9, which is not purified but reacted directly with ammonia to give the glyoxamide 10.

Glyoxamide starting material compounds substituted at the 5 position of the indole nucleus with an (acidic group) may be prepared by methods and starting materials shown in schemes 2 and 3 of Patent No. 5,654,326; the disclosure of which is incorporated herein by reference.

### IV. Uses of the Compounds of the Invention:

The indole compounds described herein are believed to achieve their beneficial therapeutic action principally by direct inhibition of mammalian (including human) sPLA₂, and not by acting as antagonists for arachidonic acid, nor other active agents below arachidonic acid in the arachidonic acid cascade, such as 5-lipoxygenases, cyclooxygenases, and etc.
One aspect of this invention relates to claim 9.
Another aspect of this invention is a use of a therapeutically effective dose of the indole compound of the invention (see, formulae I and II) for the manufacture of a medicament for treating Inflammatory Diseases such as inflammatory bowel disease, septic shock, adult respiratory distress syndrome, pancreatitis, trauma, bronchial asthma, allergic rhinitis, rheumatoid arthritis, osteoarthritis, and related diseases.

As previously noted the compounds of this invention are useful for inhibiting sPLA₂ mediated release of fatty acids such as arachidonic acid. By the term, "inhibiting" is meant the prevention or therapeutically significant reduction in release of sPLA₂ initiated fatty acids by the compounds of the invention. By "pharmaceutically acceptable" it is meant the carrier, diluent or excipient must be compatible with the other ingredients of the formulation and not deleterious to the recipient thereof.

The specific dose of a compound administered according to this invention to obtain therapeutic or prophylactic effects will, of course, be determined by the particular circumstances surrounding the case, including, for example, the compound administered, the route of administration and the condition being treated. Typical daily doses will contain a non-toxic dosage level of from about 0.01 mg/kg to about 50 mg/kg of body weight of an active compound of this invention.

Preferably compounds of the invention (per Formula I or II) or pharmaceutical formulations containing these compounds are in unit dosage form for administration to a mammal. The unit dosage form can be a capsule or tablet itself, or the appropriate number of any of these. The quantity of Active ingredient in a unit dose of composition may be varied or adjusted from about 0.1 to about 1000 milligrams or more according to the particular treatment involved. It may be appreciated that it may be necessary to make routine variations to the dosage depending on the age and condition of the patient. The dosage will also depend on the route of administration.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the indole compound of the invention together with a pharmaceutically acceptable carrier or diluent therefor. The present pharmaceutical formulations are prepared by known procedures using well known and readily available ingredients.

In making the compositions of the present invention, the Active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound. The compounds of the present invention are preferably formulated prior to administration.

For the pharmaceutical formulations any suitable carrier known in the art can be used. In such a formulation, the carrier may be a solid, liquid, or mixture of a solid and a liquid. For-example, for intravenous injection the compounds of the invention may be dissolved in at a concentration of 2 mg/ml in a 4% dextrose/0.5% Na citrate aqueous solution. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided Active ingredient. In tablets the Active ingredient is mixed with a carrier having the necessary binding properties in suitable proportions and compacted in the shape and size desired. The powders and tablets preferably contain from about 1 to about 99 weight percent of the Active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The Active ingredient can be dissolved or suspended in a pharmaceutically acceptable carrier, such as sterile water, sterile organic solvent or a mixture of both. The Active ingredient can often be dissolved in a suitable organic solvent, for instance aqueous propylene glycol. Other compositions can be made by dispersing the finely divided Active ingredient in aqueous starch or sodium carboxymethyl cellulose solution or in a suitable oil.

The following pharmaceutical formulations 1 thru 8 are illustrative only and are not intended to limit the scope of the invention in any way. "Active ingredient", refers to a compound according to Formula (I) or (II) or a pharmaceutically acceptable salt, solvate, or prodrug thereof.

### Formulation 1

Hard gelatin capsules are prepared using the following ingredients:

| | Quantity (mg/capsule) |
|---|---|
| Active ingredient | 250 |
| Starch, dried | 200 |
| Magnesium stearate | 10 |
| Total | 460 mg |

### Formulation 2

A tablet is prepared using the ingredients below:

| | Quantity (mg/tablet) |
|---|---|
| Active ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg

### Formulation 3

An aerosol solution is prepared containing the following components:

| | Weight |
|---|---|
| Active ingredient | 0.25 |
| Ethanol | 25.75 |
| Propellant 22 (Chlorodifluoromethane) | 74.00 |
| Total | 100.00 |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

### Formulation 4

Tablets, each containing 60 mg of Active ingredient, are made as follows:

| | |
|---|---|
| Active ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The Active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 5

Capsules, each containing 80 mg of Active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | 200 mg |

The Active ingredient, cellulose, starch, and magnesium stearate are blended, passed through a No. 45 mesh U.S. sieve, and filled into hard gelatin capsules in 200 mg quantities.

### Formulation 6

Suppositories, each containing 225 mg of Active ingredient, are made as follows:

| | |
|---|---|
| Active ingredient | 225 mg |
| Saturated fatty acid glycerides | 2,000 mg |
| Total | 2,225 mg |

The Active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

### Formulation 7

Suspensions, each containing 50 mg of Active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The Active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation 8

An intravenous formulation may be prepared as follows:

| | |
|---|---|
| Active ingredient | 100 mg |
| Isotonic saline | 1,000 ml |

The solution of the above ingredients generally is administered intravenously to a subject at a rate of 1 ml per minute.

All of the products of the Examples described below as well as intermediates used in the following procedures showed satisfactory nmr and IR spectra. They also had the correct mass spectral values.

### Example 1

Preparation of [[3-(2-Amino-1,2-dioxoethyl)-2-ethyl-1-(phenylmethyl)-1H-indol-4-yl]oxy]acetic acid methyl ester, a compound represented by the compound of formula (1) formula:

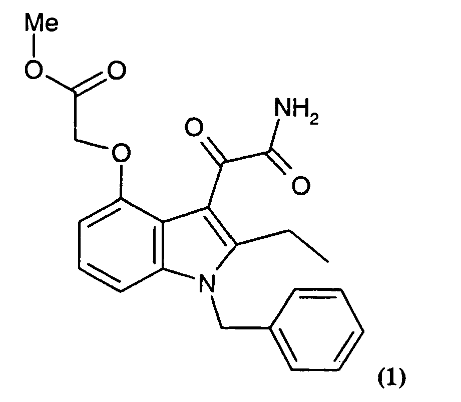

### Part A. Preparation of 2-Ethyl-4-methoxy-1H-indole.

A solution of 140 mL (0.18 mol) of 1.3M sec-butyl lithium in cyclohexane was added slowly to N-tert-butoxycarbonyl-3-methoxy-2-methylaniline (21.3g, 0.09 mol) in 250 mL of THF keeping the temperature below -40°C with a dry ice-ethanol bath. The bath was removed and the temperature allowed to rise to 0°C and then the bath replaced. After the temperature had cooled to -60°C, 18.5g (0.18 mol) of N-methoxy-N-methylpropanamide in an equal volume of THF was added dropwise. The reaction mixture was stirred 5 minutes, the cooling bath removed and stirred an additional 18 hours. It was then poured into a mixture of 300 mL of ether and 400 mL of 0.5N HCl. The organic layer was separated, washed with water, brine, dried over MgSO₄, and concentrated at reduced pressure to give 25.5g of a crude of 1-[2-(tert-butoxycarbonylamino)-6-methoxyphenyl]-2-butanone. This material was dissolved in 250 mL of methylene chloride and 50 mL of trifluoroacetic acid and stirred for a total of 17 hours. The mixture was concentrated at reduced pressure and ethyl acetate and water added to the remaining oil. The ethyl acetate was separated, washed with brine, dried (MgSO₄) and concentrated. The residue was chromatographed three times on silica eluting with 20% EtOAc/hexane to give 13.9g of 2-ethyl-4-methoxy-1H-indole.

| Analyses for C₁₁H₁₃NO: | | | |
|---|---|---|---|
| Calculated: | C, 75.40; | H, 7.48; | N, 7.99 |
| Found: | C, 74.41; | H, 7.64; | N, 7.97. |

### Part B. Preparation of 2-Ethyl-4-methoxy-1-(phenylmethyl)-1H-indole.

2-Ethyl-4-methoxy-1H-indole (4.2g, 24 mmol) was dissolved in 30 mL of DMF and 960mg (24 mmol) of 60% NaH/minerial oil was added. After 1.5 hours, 2.9 mL(24 mmol) of benzyl bromide was added. After 4 hours, the mixture was diluted with water and extracted twice with ethyl acetate. The combined ethyl acetate was washed with brine, dried (MgSO₄) and concentrated at reduced pressure. The residue was chromatographed on silica gel and eluted with 20% EtOAc/hexane to give 3.1g (49% yield) of 2-ethyl-4-methoxy-1-(phenylmethyl)-1H-indole.

### Part C. Preparation of 2-Ethyl-4-hydroxy-1-(phenylmethyl)-1H-indole.

3.1g (11.7 mmol) of 2-ethyl-4-methoxy-1-(phenylmethyl)-1H-indole was O-demethylated by treating it with 48.6 mL of 1M BBr₃ in methylene chloride with stirring at room temperature for 5 hours, followed by concentration at reduced pressure. The residue was dissolved in ethyl acetate, washed with brine and dried (MgSO₄). After concentrating at reduced pressure, the residue was chromatographed on silica gel eluting with 20% EtOAc/hexane to give 1.58g (54% yield) of 2-ethyl-4-hydroxy-1-(phenylmethyl)-1H-indole, mp, 86-90°C.

| Analyses for C₁₇H₁₇NO: | | | |
|---|---|---|---|
| Calculated: | C, 81.24; | H, 6.82; | N, 5.57 |
| Found: | C, 81.08; | H, 6.92; | N, 5.41. |

### Part D. Preparation of [[2-Ethyl-1-(phenylmethyl)-1H-indol-4-yl]oxy]acetic acid methyl ester.

2-ethyl-4-hydroxy-1-(phenylmethyl)-1H-indole (1.56g, 6.2 mmol) was added to a mixture of 248mg (6.2 mmol) of 60% NaH/mineral oil in 20mL DMF and stirred for 0.67 hour.

Then 0.6 mL(6.2 mmol) of methyl bromoacetate was added and stirring was continued for 17 hours. The mixture was diluted with water and extracted with ethyl acetate. The ethyl acetate solution was washed with brine, dried (MgSO₄), and concentrated at reduced pressure. The residue was chromatographed on silica gel eluting with 20% EtOAc/hexane, to give 1.37g (69% yield) of [[2-ethyl-1-(phenylmethyl)-1H-indol-4-yl]oxy]acetic acid methyl ester, 89-92°C.

| Analyses for C₂₀H₂₁NO₃: | | | |
|---|---|---|---|
| Calculated: | C, 74.28; | H, 6.55; | N, 4.33 |
| Found: | C, 74.03; | H, 6.49; | N, 4.60. |

### Part E. Preparation of [[3-(2-Amino-1,2-dioxoethyl)-2-ethyl-1-(phenylmethyl)-1H-indol-4-yl]oxy]acetic acid methyl ester.

Oxalyl chloride (0.4 mL, 4.2 mmol) was added to 1.36g (4.2 mmol) of [[2-ethyl-1-(phenylmethyl)-1H-indol-4-yl]oxy]acetic acid methyl ester in 10 mL of methylene chloride and the mixture stirred for 1.5 hours. The mixture was concentrated at reduced pressure and residue taken up in 10 mL of methylene chloride. Anhydrous ammonia was bubbled in for 0.25 hours, the mixture stirred for 1.5 hours and evaporated at reduced pressure. The residue was stirred with 20 mL of ethyl acetate and the mixture filtered. The filtrate was concentrated to give 1.37g of a mixture of [[3-(2-amino-1,2-dioxoethyl)-2-ethyl-1-(phenylmethyl)-1H-indol-4-yl]oxy]acetic acid methyl ester and ammonium chloride. This mixture melted at 172-187°C.

### Example 2

### (indol-3-oxime amide starting material)

### 2-[[3-[[2-(Aminooxo)-1-(N-hydroxyimino)]ethyl]-2-ethyl-1-(phenylmethyl)-1H-indol-4-yl]oxy]acetic acid.

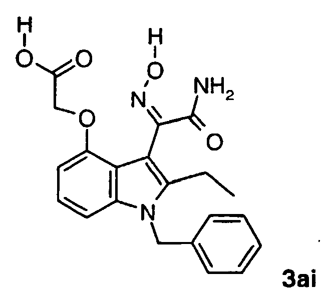

### A. Preparation of 2-[[3-[[2-(Aminooxo)-1-(N-hydroxyimino)]ethyl]-2-ethyl-1-(phenylmethyl)-1H-indol-4-yl]oxy]acetic acid methyl ester.

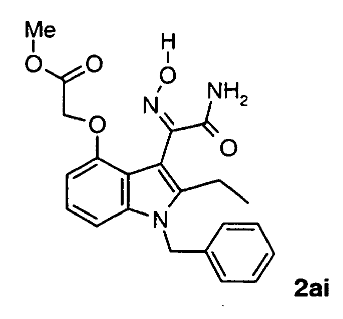

A stirred mixture of 1 (600 mg, 1.52 mmol) and hydroxylamine hydrochloride (528 mg, 7.60 mmol) in THF (4 mL)/CH₃OH (4 mL) was heated at 55 °C for 8 h. After concentration at ambient temperature, the residue was chromatographed on silica (gradient 0-40% EtOAc in CH₂Cl₂) to give the title compound 2ai (285 mg) as a white solid in 46% yield. IR (CHCl₃) 3510, 3415, 1757, 1667 cm⁻¹; ¹H-NMR (CDCl₃) δ 1.17 (t, *J* = 7.5 Hz, 3H), 2.84 (q, *J* = 7.5 Hz, 2H), 3.81 (s, 3H), 4.73 (s, 2H), 5.36 (s, 2H), 5.67 (br s, 1H), 6.31 (br s, 1H), 6.41 (d, *J* = 7.8 Hz, 1H), 6.87 (d, *J* = 8.2 Hz, 1H), 6.98-7.07 (m, 3H), 7.23-7.32 (m, 3H); ESIMS *m*/*e* 410 (M⁺+1).

| Elemental Analyses for C₂₂H₂₃N₃O₅·0.30(H₂O) : | | | |
|---|---|---|---|
| Calculated: | C, 63.70; | H, 5.73; | N, 10.13; |
| Found: | C, 63.68; | H, 5.62; | N, 10.20. |

### Example 3

### N-[2-[[3-(Aminooxoacetyl)-2-ethyl-1-(phenylmethyl)-1H-indol-4-yl]oxy]acetyl]glycine

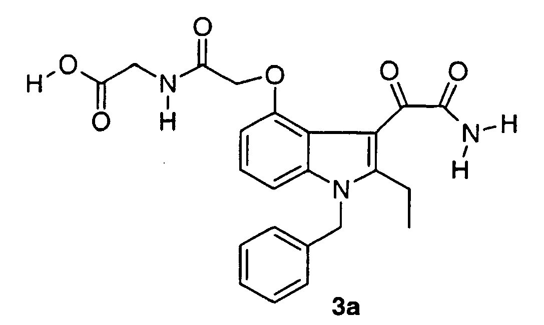

### A. Preparation of N-[2-[[3-(Aminooxoacetyl)-2-ethyl-1-(phenylmethyl)-1H-indol-4-yl]oxy]acetyl]glycine methyl ester

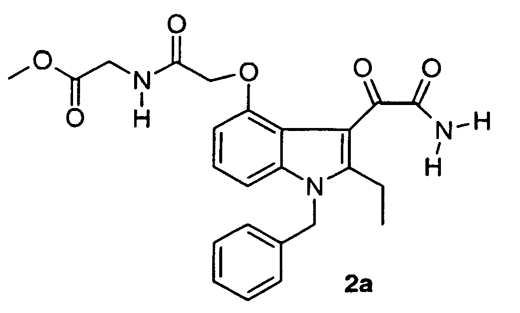

To a solution of 1 (0.100 g, 0.249 mmol) in 2 mL DMF was added collidine (0.069 mL, 0.523 mmol), methyl glycine hydrochloride (0.0313 g,0.249 mmol), and benzotriazolyl-*N-*oxy-tris(dimethylamino)phosphonium hexafluorophosphate (0.115 g, 0.261) sequentially at room temperature. After 2.5 hrs. the reaction mixture was concentrated *in vacuo* to near dryness, then it was taken up in CH₂Cl₂, chromatographed on a silica gel column (gradient 20-40% THF in CH₂Cl₂) and dried in an 80°C vacuum oven to give 0.0768 g of 2a as a yellow solid in 68% yield. ¹H NMR (DMSO-d₆) δ 1.04 (t, *J* = 6.8 Hz, 3H), 2.90 (br q, *J* = 6.8 Hz, 2H), 3.57 (s, 3H), 3.88 (d, *J* = 5.5 Hz, 2H), 4.57 (s, 2H), 5.51 (s, 2H), 6.59 (d, *J* = 5.6 Hz, 1H), 7.01-7.08 (m 4H), 7.19-7.30 (m, 3H), 7.55 (s, 1H), 7.99 (s, 1H), 8.40 (t, *J* = 5.5 Hz, 1H).

### B. Preparation of N-[2-[[3-(Aminooxoacetyl)-2-ethyl-1-(phenylmethyl)-1H-indol-4-yl]oxy]acetyl]glycine

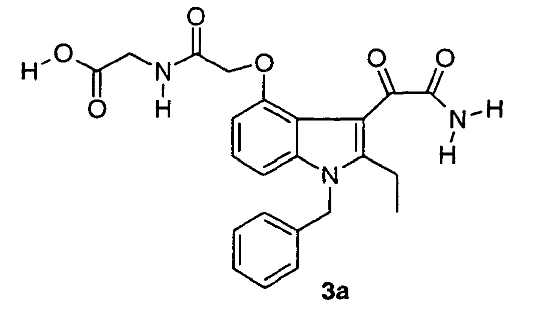

To a solution of 2a (0.035 g, 0.078 mmol) in 1 mL THF, 1 mL MeOH and 0.25 mL distilled H₂O was added 4.17N LiOH (0.093 mL, 0.388 mmol) at room temperature. After 2 hrs. the reaction mixture was acidified with 5N HCl (0.093 mL, 0.465 mmol) and concentrated *in vacuo.* The residue was taken up in CH₂Cl₂ then rapidly triturated with hexanes to give a yellow suspension which was filtered and dried in an 80°C vacuum oven to give 0.0336 g of 3a as a yellow solid in 99% yield. ¹H NMR (DMSO-d₆) δ 1.04 (t, *J* = 5.9 Hz, 3H), 2.90 (br q, *J =* 5.9 Hz, 2H), 3.80 (d, *J =* 4.8 Hz, 2H), 4.56 (s, 2H), 5.51 (s, 2H), 6.62 (d, J = 5.8 Hz, 1H), 7.01-7.28 (m, 7H), 7.54 (s, 1H), 7.99 (s, 1H), 8.31 (t, J = 4.8 Hz, 1H), 12.25-12.75 (br s, 1H).

### Example 4

### N-[2-[[3-(Aminooxoacetyl)-2-ethyl-1-(phenylmethyl)-1H-indol-4-yl]oxy]acetyl]-L-alanine

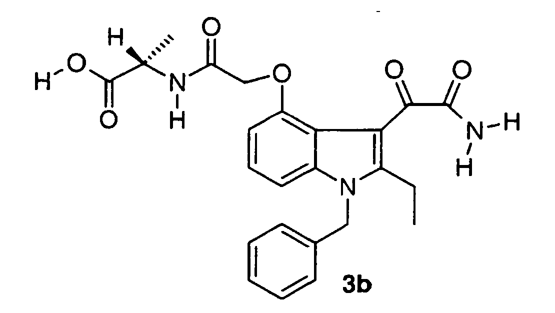

### A. Preparation of N-[2-[[3-(Aminooxoacetyl)-2-ethyl-1-(phenylmethyl)-1H-indol-4-yl]oxy]acetyl]-L-alanine methyl ester

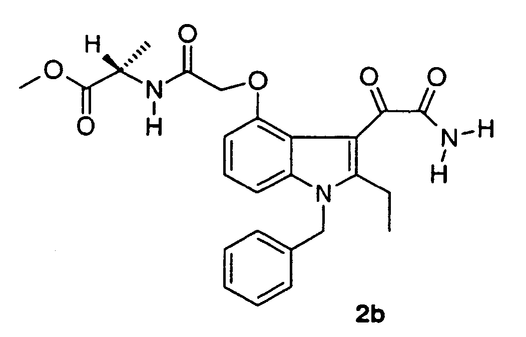

Following the experimental procedure as described for 2a, 2b was obtained as a yellow solid in 65% yield.
¹H NMR (DMSO-d₆) δ 1.04 (t, *J* = 7.2 Hz, 3H), 1.29 (d, *J* = 7.3 Hz, 3H), 2.91 (br q, *J* = 7.2 Hz, 2H), 3.54 (s, 3H), 4.29 (qd, *J* = 7.3, 6.8 Hz, 1H), 4.55 (s, 2H), 5.51 (s, 2H), 6.57 (m, 1H), 6.99 (d, J = 7.4 Hz, 2H), 7.07-7.08 (m, 2H), 7.21-7.31 (m, 3H), 7.56 (s, 1H), 8.05 (s, 1H), 8.40 (d, *J* = 6.8 Hz, 1H).

### B. Preparation of N-[2-[[3-(Aminooxoacetyl)-2-ethyl-1-(phenylmethyl)-1H-indol-4-yl]oxy]acetyl]-L-alanine

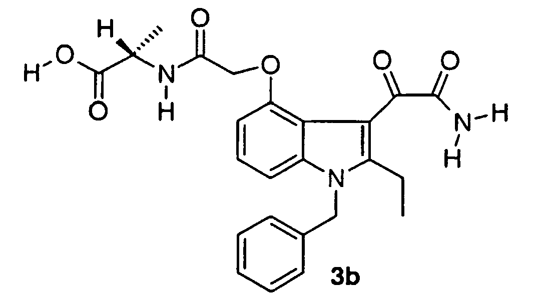

Following the experimental procedure as described for preparing compound 3a, compound 3b, was obtained as a yellow solid in 89% yield. ¹H NMR (DMSO-d₆) δ 1.04 (t, *J* = 7.2 Hz, 3H), 1.29 (d, *J* = 7.3 Hz, 3H), 2.91 (br q, *J* = 7.2 Hz, 2H), 4.22 (td, *J* = 7.2, 7.1 Hz, 1H), 4.54(s, 2H), 5.51 (s, 2H), 6.60 (d, *J* = 6.3 Hz, 1H), 7.00-7.09 (m, 4H), 7.21-7.30 (m, 3H), 7.53 (s, 1H), 8.03 (s, 1H), 8.31 (d, *J* = 7.1 Hz, 1H), 12.75-12.84 (br s, 1H).

### Example 5

### N-[2-[[3-(Aminooxoacetyl)-2-ethyl-1-(phenylmethyl)-1H-indol-4-yl]oxy]acetyl]-L-leucine

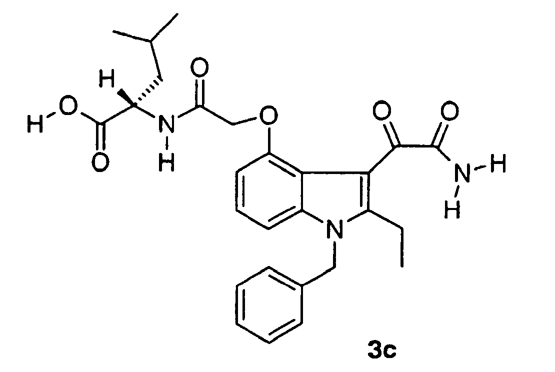

### A. Preparation of N-[2-[[3-(Aminooxoacetyl)-2-ethyl-1-(phenylmethyl)-1H-indol-4-yl]oxy]acetyl]-L-leucine methyl ester

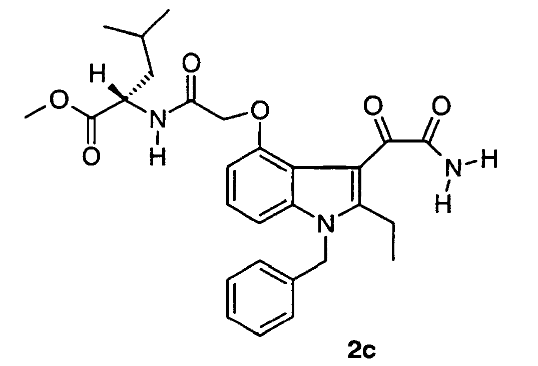

Following the experimental procedure as described for 2a, 2c was obtained as a yellow solid in 98% yield. ¹H NMR (DMSO-d₆) δ 0.67 (d, *J* = 5.5 Hz, 3H), 0.72 (d, *J* = 5.7 Hz, 3H), 1.05 (t, *J* = 7.2 Hz, 3H), 1.30-1.50 (m, 2H), 1.51-1.64 (m, 1H), 2.91 (br q, *J* = 7.2 Hz, 2H), 3.55 (s, 3H), 4.20-4.27 (m, 1H), 4.57 (s, 2H), 5.52 (s, 2H), 6.53-6.56 (m, 1H), 6.97-7.08 (m, 4H), 7.21-7.29 (m, 3H), 7.56 (s, 1H), 8.07 (s, 1H), 8.37 (d, *J* = 7.3 Hz, 1H).

### B. Preparation of N-[2-[[3-(Aminooxoacetyl)-2-ethyl-1-(phenylmethyl)-1H-indol-4-yl]oxy]acetyl]-L-leucine

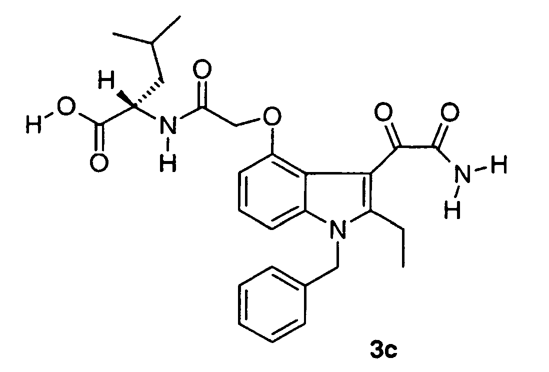

Following the experimental procedure as described for 3a, 3c was obtained as a yellow solid in 75% yield. ¹H NMR (DMSO-d₆) δ 0.76 (d, *J =* 5.7 Hz, 3H), 0.78 (d, *J* = 6.1 Hz, 3H), 1.21 (t, *J* = 7.3 Hz, 3H), 1.39-1.43 (m, 1H), 1.69 (t, *J* = 7.3 Hz, 2H), 2.96 (br q, *J* = 7.3 Hz, 2H), 4.57-4.65 (m, 1H), 4.69 (d, *J* = 16.0 Hz, 1H), 4.78 (d, *J* = 16.0 Hz, 1H), 5.38 (s, 2H), 6.59 (d, *J* = 8.0 Hz, 1H), 6.89 (d, *J* = 8.2 Hz, 1H), 6.95-7.12 (m, 5H), 7.26-7.32 (m, 3H), 8.17 (d, *J* = 8.2 Hz, 1H).

### Example 6

### N-[2-[[3-(Aminooxoacetyl)-2-ethyl-1-(phenylmethyl)-1H-indol-4-yl]oxy]acetyl]-L-aspartic acid

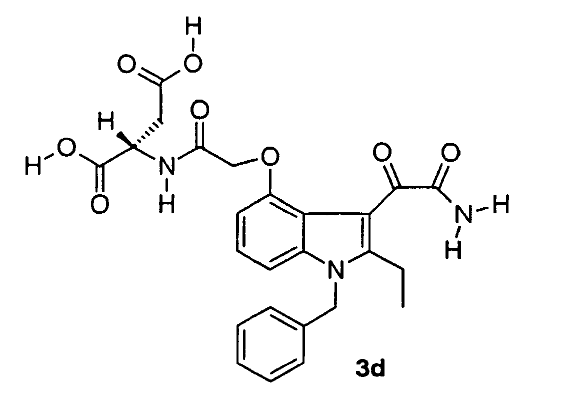

### A. Preparation of N-[2-[[3-(Aminooxoacetyl)-2-ethyl-1-(phenylmethyl)-1H-indol-4-yl]oxy]acetyl]-L-aspartic acid dimethyl ester

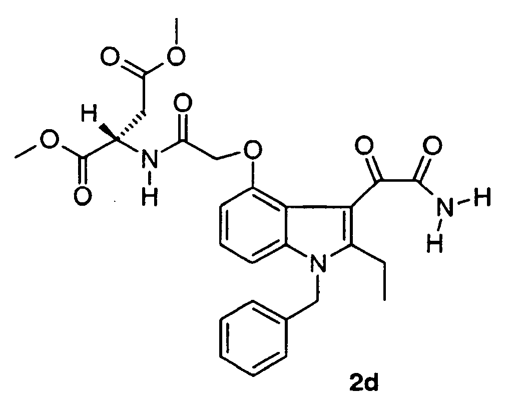

Following the experimental procedure as described for 2a, 2d was obtained as a yellow solid in 88% yield. ¹H NMR (DMSO-d₆) δ 1.04 (t, *J =* 7.3 Hz, 3H), 2.72 (dd, *J* = 16.6, 7.1 Hz, 1H), 2.83 (dd, *J* = 16.7, 7.1 Hz, 1H), 2.90 (br q, *J* = 7.3 Hz, 2H), 3.49 (s, 3H), 3.55 (s, 3H), 4.54 (s, 2H), 4.66 (m, 1H), 5.51 (s, 2H), 6.54 (m, 1H), 6.97-7.09 (m, 4H), 7.21-7.30 (m, 3H), 7.50 (s, 1H), 7.97 (s, 1H), 8.52 (d, *J* = 7.9 Hz, 1H).

### B. Preparation of N-[2-[[3-(Aminooxoacetyl)-2-ethyl-1-(phenylmethyl)-1H-indol-4-yl]oxy]acetyl]-L-aspartic acid

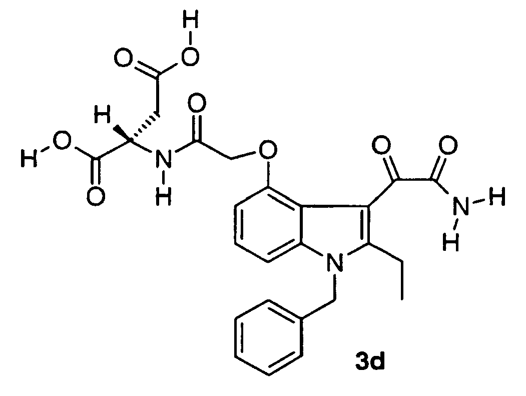

Following the experimental procedure as described for 3a, 3d was obtained as a yellow solid in 99% yield. ¹H NMR (DMSO-d₆) δ 1.04 (t, *J* = 7.2 Hz, 3H), 2.52-2.76 (m, 2H), 2.90 (br q, *J* = 7.2 Hz, 2H), 4.53 (s, 2H), 4.53-4.60 (m, 1H), 5.50 (s, 2H), 6.59 (d, *J* = 7.2 Hz, 1H), 6.98-7.09 (m, 4H), 7.19-7.30 (m, 3H), 7.47 (s, 1H), 7.94 (s, 1H), 8.33 (d, *J* = 7.8 Hz, 1H), 12.40-13.20 (br s, 2H).

### Example 7

### N-[2-[[3-(Aminooxoacetyl)-2-ethyl-1-(phenylmethyl)-1H-indol-4-yl]oxy]acetyl]-L-phenylalanine

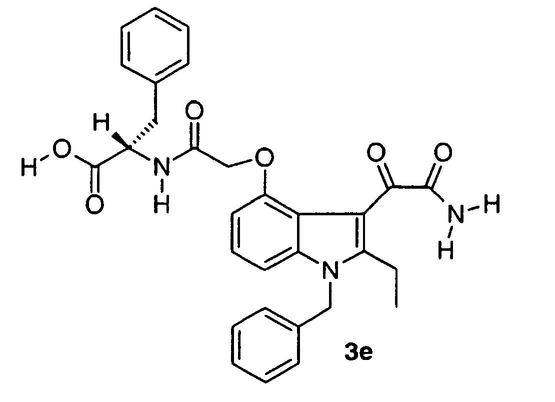

### A. Preparation of N-[2-[[3-(Aminooxoacetyl)-2-ethyl-1-(phenylmethyl)-1H-indol-4-yl]oxy]acetyl]-L-phenylalanine methyl ester

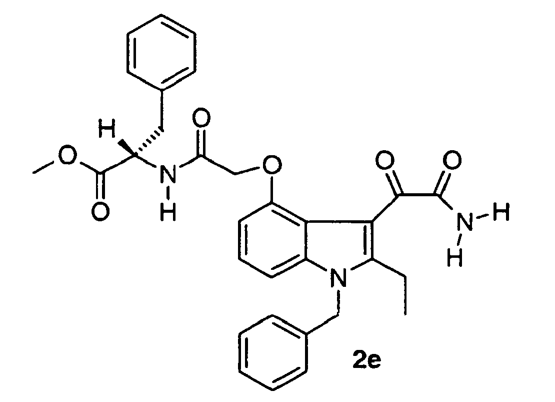

Following the experimental procedure as described for 2a, 2e was obtained as a yellow solid in 68% yield. ¹H NMR (DMSO-d₆) δ 1.06 (t, *J* = 7.2 Hz, 3H), 2.88-3.03 (m, 4H), 3.54 (s, 3H), 4.47-4.50 (m, 1H), 4.50 (s, 2H), 5.52 (s, 2H), 6.41 (d, *J* = 7.7 Hz, 1H), 6.98-7.11 (m, 9H), 7.21-7.30 (m, 3H), 7.47 (s, 1H), 8.06 (s, 1H), 8.52 (d, *J* = 7.7 Hz, 1H).

### B. Preparation of N-[2-[[3-(Aminooxoacetyl)-2-ethyl-1-(phenylmethyl)-1H-indol-4-yl]oxy]acetyl]-L-phenylalanine

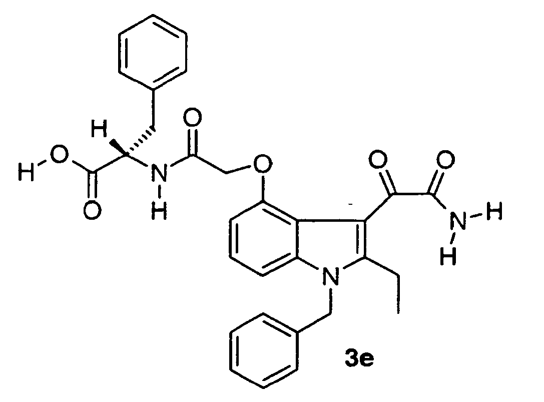

Following the experimental procedure as described for 3a, 3e was obtained as a yellow solid in 93% yield. ¹H NMR (DMSO-d₆) δ 1.04 (t, *J* = 7.1 Hz, 3H), 2.85-3.12 (m, 4H), 4.17-4.26 (m, 1H), 4.54 (s, 2H), 5.51 (s, 2H), 6.59 (d, *J* = 6.4 Hz, 1H), 6.98-7.09 (m, 9H), 7.19-7.30 (m, 3H), 7.53 (s, 1H), 8.03 (s, 1H), 8.30 (d, *J*= 7.0 Hz, 1H), 12.50 (br s, 1H).

### Example 8

### [2-[[3-(Aminooxoacetyl)-2-ethyl-1-(phenylmethyl)-1H-indol-4-yl]oxy]acetamido]malonic acid

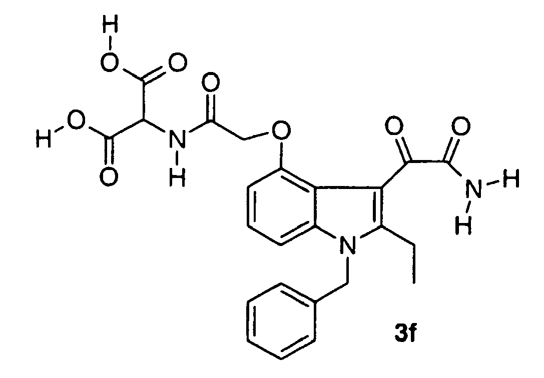

### A. Preparation of [2-[[3-(Aminooxoacetyl)-2-ethyl-1-(phenylmethyl)-1H-indol-4-yl]oxy]acetamido]malonic acid dimethyl ester

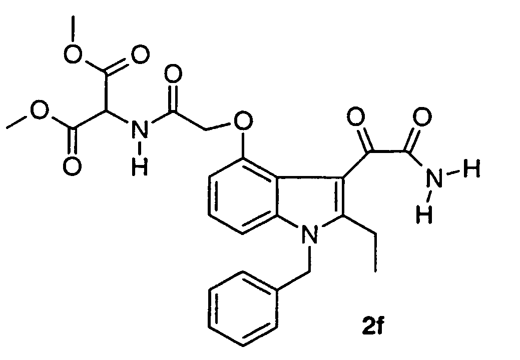

Following the experimental procedure as described for 2a, 2f was obtained as a yellow solid in 98% yield. ¹H NMR (DMSO-d₆) δ 1.04 (t, *J* = 7.3 Hz, 3H), 2.90 (br q, *J* = 7.3 Hz, 2H), 3.64 (s,6H), 4.63 (s, 2H), 5.16 (d, *J* = 7.1 Hz, 1H), 5.51 (s, 2H), 6.54-6.56 (m, 1H), 6.98-7.09 (m, 4H), 7.21-7.30 (m, 3H), 7.43 (s, 1H), 7.88 (s, 1H), 8.90 (d, *J* = 7.2 Hz, 1H).

### B. Preparation of [2-[[3-(Aminooxoacetyl)-2-ethyl-1-(phenylmethyl)-1H-indol-4-yl]oxy]acetamido]malonic acid

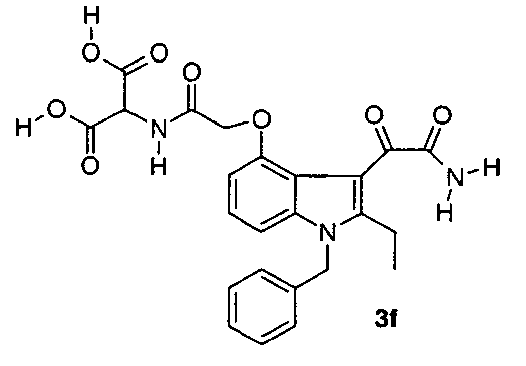

Following the experimental procedure as described for 3a, 3f was obtained as a yellow solid in 99% yield. ¹H NMR (DMSO-d₆) δ 1.04 (t, *J* = 6.9 Hz, 3H), 2.89 (br q, *J* = 7.3 Hz, 2H), 4.62 (s, 2H), 4.91 (d, *J* = 7.2 Hz, 1H), 5.50 (s, 2H), 6.57 (d, *J =* 7.2 Hz, 1H), 6.98-7.09 (m, 4H), 7.18-7.30 (m, 3H), 7.37 (s, 1H), 7.83 (s, 1H), 8.55 (d, *J =* 7.2 Hz, 1H), 12.30-13.00 (br s, 2H).

### Example 9

### N-[2-[[3-(Aminooxoacetyl)-2-ethyl-1-(phenylmethyl)-1H-indol-4-yl]oxy]acetyl]-L-valine

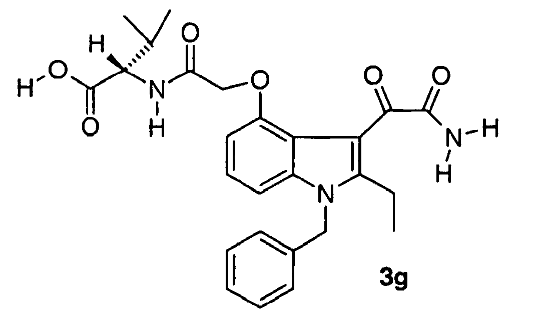

### A. Preparation of N-[2-[[3-(Aminooxoacetyl)-2-ethyl-1-(phenylmethyl)-1H-indol-4-yl]oxy]acetyl]-L-valine methyl ester

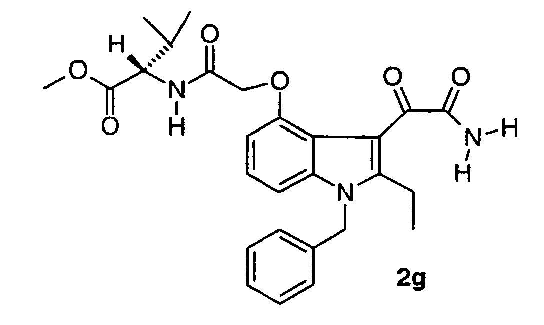

Following the experimental procedure as described for 2a, 2g was obtained as a yellow solid in 96% yield. ¹H NMR (DMSO-d₆) δ 0.71 (d, *J* = 6.8 Hz, 3H), 0.74 (d, *J* = 7.0 Hz, 3H), 1.05 (t, *J* = 7.2 Hz 3H), 1.99-2.05 (m, 1H), 2.90 (br q, *J* = 7.2 Hz, 2H), 3.54 (s, 3H), 4.11 (br t, *J* = 7.0 Hz, 1H), 4.60 (s, 2H), 5.52 (s, 2H), 6.52 (d, *J* = 4.4 Hz, 1H), 6.95 (d, *J* = 7.2 Hz, 2H), 7.06 (br s, 2H), 7.18-7.29 (m, 3H), 7.52 (s, 1H), 8.04 (s, 1H), 8.20 (d, *J* = 7.8 Hz, 1H).

### B. Preparation of N-[2-[[3-(Aminooxoacetyl)-2-ethyl-1-(phenylmethyl)-1H-indol-4-yl]oxy]acetyl]-L-valine

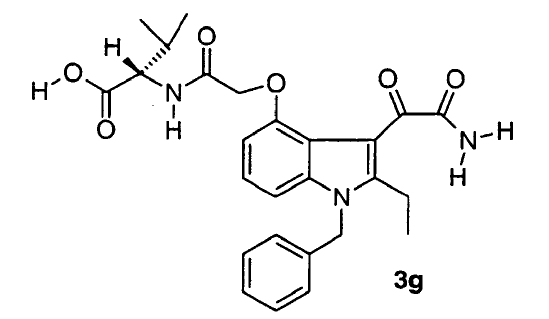

Following the experimental procedure as described for 3a, 3g was obtained as a yellow solid in 94% yield. ¹H NMR (DMSO-d₆) δ 0.71 (d, *J* = 6.9 Hz, 3H), 0.75 (d, *J* = 6.8 Hz, 3H), 1.04 (t, *J* = 7.3 Hz 3H), 2.01-2.07 (m, 1H), 2.90 (br q, *J* = 7.3 Hz, 2H), 4.09 (br dd, *J* = 7.9, 6.2 Hz, 1H), 4.60 (s, 2H), 5.51 (s, 2H), 6.54 (d, *J* = 6.1 Hz, 1H), 6.95 (d, *J* = 7.3 Hz, 2H), 6.99-7.08 (m, 2H), 7.18-7.29 (m, 3H), 7.49 (s, 1H), 8.01 (s, 1H), 8.08 (d, *J* = 8.2 Hz, 1H), 12.63 (br s, 1H).

### Example 10

### N-[2-[[3-(Aminooxoacetyl)-2-ethyl-1-(phenylmethyl)-1H-indol-4-yl]oxy]acetyl]-L-isoleucine

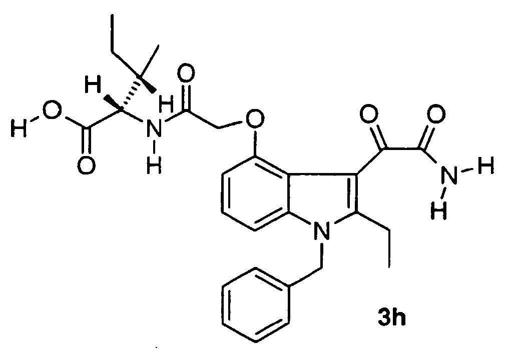

### A. Preparation of N-[2-[[3-(Aminooxoacetyl)-2-ethyl-1-(phenylmethyl)-1H-indol-4-yl]oxy]acetyl]-L-isoleucine methyl ester

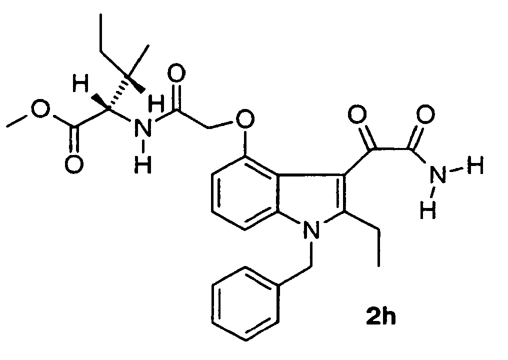

Following the experimental procedure as described for 2a, 2h was obtained as a yellow solid in 73% yield. ¹H NMR (DMSO-d₆) δ 0.64-0.71 (m, 6H), 0.99-1.08 (m, 4H), 1.21-1.26 (m, 1H), 1.76-1.80 (m, 1H), 2.91 (br q, *J* = 7.4 Hz, 2H), 3.53 (s, 3H), 4.15 (br t, *J* = 7.2 Hz, 1H), 4.60 (s, 2H), 5.52 (s, 2H), 6.52 (m, 1H), 6.96 (d, *J* = 7.2 Hz, 2H), 7.02-7.07 (m, 2H), 7.18-7.29 (m, 3H), 7.53 (s, 1H), 8.04 (s, 1H), 8.23 (d, *J* = 7.7 Hz, 1H).

### B. Preparation of N-[2-[[3-(Aminooxoacetyl)-2-ethyl-1-(phenylmethyl)-1H-indol-4-yl]oxy]acetyl]-L-isoleucine

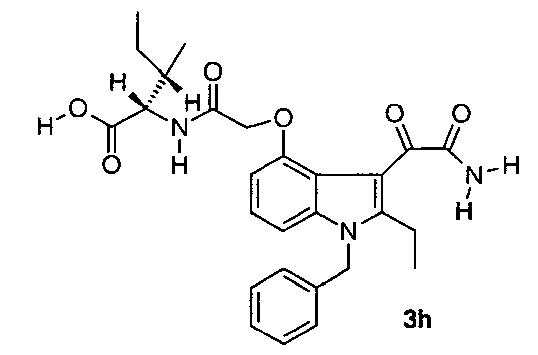

Following the experimental procedure as described for 3a, 3h was obtained as a yellow solid in 92% yield. ¹H NMR (DMSO-d₆) δ 0.64-0.84 (m, 6H), 1.04 (t, *J =* 7.2 Hz, 3H), 1.21-1.28 (m, 2H), 1.76-1.80 (m, 1H), 2.91 (br q, *J* = 7.2 Hz, 2H), 4.12 (br t, *J* = 7.3 Hz, 1H), 4.59 (s, 2H), 5.51 (s, 2H), 6.55 (d, J = 6.4 Hz, 1H), 6.96 (d, *J* = 7.2 Hz, 2H), 7.01-7.08 (m, 2H), 7.21-7.29 (m, 3H), 7.51 (s, 1H), 8.01 (s, 1H), 8.11 (d, *J* = 7.4 Hz, 1H), 12.40-12.65 (br s, 1H).

### Assay

The following chromogenic assay procedure was used to identify and evaluate inhibitors of recombinant human secreted phospholipase A₂. The assay described herein has been adapted for high volume screening using 96 well microtiter plates. A general description of this assay method is found in the article, "Analysis of Human Synovial Fluid Phospholipase A₂ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992 (the disclosure of which is incorporated herein by reference):

### Reagents:

REACTION BUFFER -
CaCl₂·2H₂O (1.47 g/L)
KCl (7.455 g/L)
Bovine Serum Albumin (fatty acid free) (1 g/L)
(Sigma A-7030, product of Sigma
Chemical Co., St. Louis MO, USA)
TRIS HCl (3.94 g/L)
pH 7.5 (adjust with NaOH)

ENZYME BUFFER -
0.05 NaOAc.3H₂O, pH 4.5
0.2 NaCl
Adjust pH to 4.5 with acetic acid
DTNB - 5,5'-dithiobis-2-nitrobenzoic acid

RACEMIC DIHEPTANOYL THIO - PC
racemic 1,2-bis(heptanoylthio)-1,2-dideoxy-*sn-*glycero-3-phosphorylcholine
TRITON X-100^{™} prepare at 6.249 mg/ml in
reaction buffer to equal 10uM.

REACTION MIXTURE -
A measured volume of racemic dipheptanoyl thio PC supplied in chloroform at a concentration of 100 mg/ml is taken to dryness and redissolved in 10 millimolar
TRITON X-100^{™} nonionic detergent aqueous solution.
Reaction Buffer is added to the solution, then DTNB to give the Reaction Mixture.
The reaction mixture thus obtained contains 1mM diheptanoly thio-PC substrate, 0.29 mm Triton X-100^{™} detergent, and 0.12 mm DTMB in a buffered aqueous solution at pH 7.5.

### Assay Procedure:

1. Add 0.2 ml reaction mixture to all wells;
2. Add 10 ul test compound (or solvent blank) to appropriate wells, mix 20 seconds;
3. Add 50 nanograms of sPLA₂ (10 microliters) to appropriate wells;
4. Incubate plate at 40 °C for 30 minutes;
5. Read absorbance of wells at 405 nanometers with an automatic plate reader.

All compounds were tested in triplicate. Typically, compounds were tested at a final concentration of 5 ug/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, IC₅₀ values were determined. Typically, the IC₅₀ values (see, Table I, below) were determined by diluting test compound serially two-fold such that the final concentration in the reaction ranged from 45 ug/mL to 0.35 ug/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of IC₅₀ values. IC₅₀ were determined by plotting log concentration versus inhibition values in the range from 10-90% inhibition.
Results of Human Secreted Phospholipase A₂ Inhibition Tests

**Table**

| Compound No. from Examples 3-10 | Inhibition of human secreted PLA₂ IC50 ± mean deviation |
|---|---|
| | (3-4 tests)(nM) |
| 1 | 49 |
| 2A | 529 |
| 2B | 533 |
| 2C | 82 |
| 2D | 874 |
| 2E | 666 |
| 2F | 698 |
| 2G | 283 |
| 2H | 166 |
| 3A | 71 |
| 3B | 59 |
| 3C | 28 |
| 3D | 132 |
| 3E | 64 |
| 3F | 44.7 |
| 3G | 36.4 |
| 3H | 25.1 |

The compound of Example 1 is highly active in inhibiting sPLA₂.

While the present invention has been illustrated above by certain specific embodiments, it is not intended that these specific examples should limit the scope of the invention as described in the appended claims.

## Claims

1. An indole compound represented by the formula (I), or a pharmaceutically acceptable salt, solvate, or prodrug derivative selected from the methyl, ethyl, propyl, isopropyl, n-butyl, isobutyl, tert-butyl, morpholinoethyl, and N,N-diethylglycolamido esters thereof; 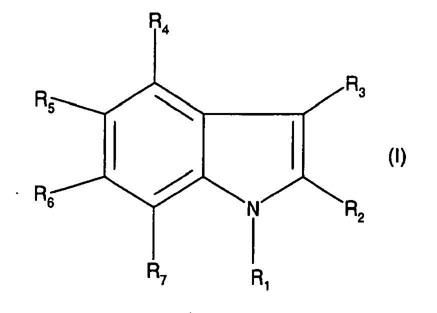 wherein ;
R₁ is the group -(L₁)-R₁₁; where, -(L₁)- is selected from a group represented by the formulae (Ia), (Ib), (Ic), (Id), (Ie), and (If): 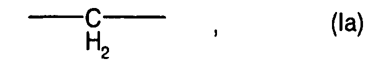
-O- , (Ib)
-S- , (Ic) 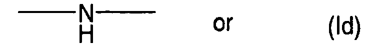 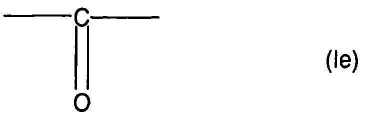
or 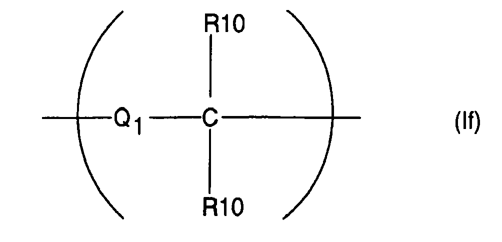 where Q₁ is a bond or any of the divalent groups Ia, Ib, Ic, Id, and Ie and R₁₀ is independently -H, C₁₋₈ alkyl, C₁₋₈ haloalkyl or C₁₋₈ alkoxy and where R₁₁ is a substituted or unsubstituted carbocyclic radical selected from the group consisting of C₅-C₁₄ cycloalkyl, C₅-C₁₄ cycloalkenyl, phenyl, spiro[5.5]undecanyl, naphthyl, norbornanyl, bicycloheptadienyl, tolulyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (a): 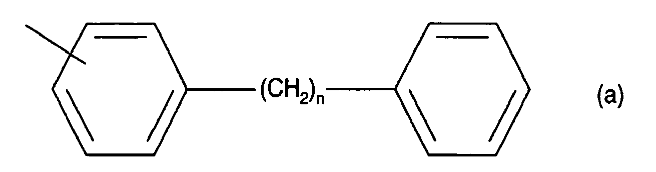 where n is a number from 1 to 8;
R₂ is hydrogen, C₁-C₄ alkyl, C₂-C₄ alkenyl, -O-(C₁-C₃ alkyl), -S-(C₁-C₃ alkyl), C₃-C₄ cycloalkyl, -CF₃, halo, -NO₂, -CN, or -SO₃;
R₃ is -(L₃)- Z, where -(L₃)- is a bond and Z is selected from a group represented by the formulae, 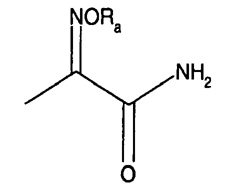 wherein Rₐ is hydrogen, methyl, ethyl, propyl, isopropyl, phenyl or benzyl, 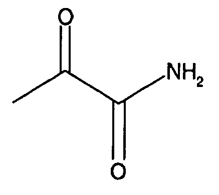 or 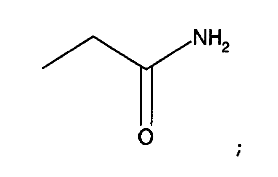
R₄ is the group, -(L_{c})-(acylamino acid group); wherein -(L_{c})- is a divalent group selected from,
⁅-O-CH₂-⁆,
⁅-S-CH₂-⁆, 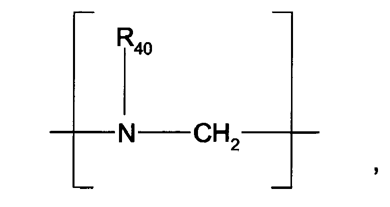 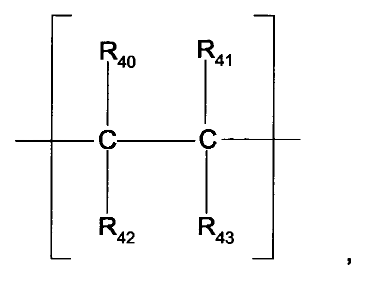
or 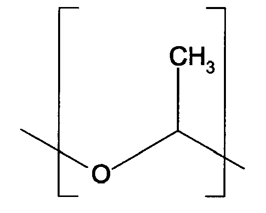 where R₄₀, R₄₁, R₄₂, and R₄₃ are each independently selected from hydrogen, C₁-C₈ alkyl and wherein the (acylamino acid group) is: 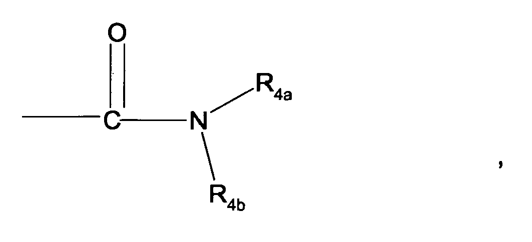 and R^{4a} is selected from the group consisting of H, (C₁-C₆)alkyl, (C₁-C₆)alkoxy and aryl; and wherein NR^{4b} is an amino acid residue
selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine *1*-proline, and *d*-proline;
R₅ is hydrogen; and
R₆ and R₇ are hydrogen;
provided that the compound 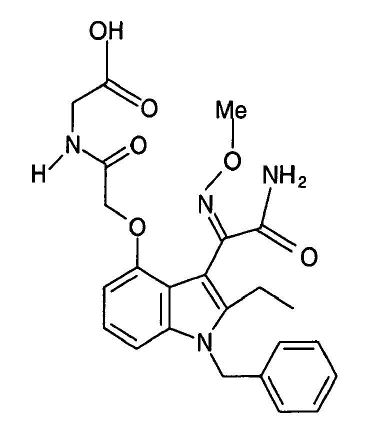 is excluded.

2. The compound of claim 1 wherein for R₃, Z is the group represented by the formula; 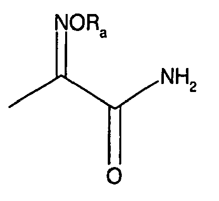 and Rₐ is hydrogen.

3. The compound of claim 1 wherein the linking group -(L₁)- of R₁ is -(CH₂)- or -(CH₂-CH₂)-.

4. The compound of Claim 1 wherein for R₁ the combined group -(L₁)-R₁₁ is selected from the groups; 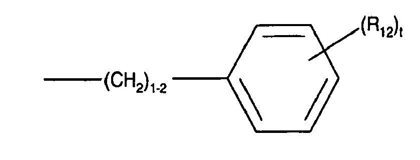 or 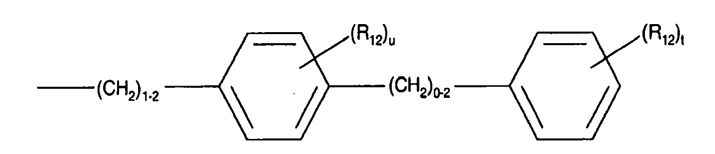 where R₁₂ is a radical independently selected from halo, C₁-C₁₀ alkyl, C₁-C₁₀ alkoxy, -S-(C₁-C₁₀ alkyl), C₁-C₁₀ haloalkyl, and C₁-C₁₀ hydroxyalkyl and t is a number from 0 to 5 and u is a number from 0 to 4.

5. An indole compound represented by the formula (II), or a pharmaceutically acceptable salt, solvate, or prodrug selected from the methyl, ethyl, propyl, isopropyl, n-butyl, isobutyl, tert-butyl, morpholineothyl, and N,N-diethylglycolamido esters thereof; 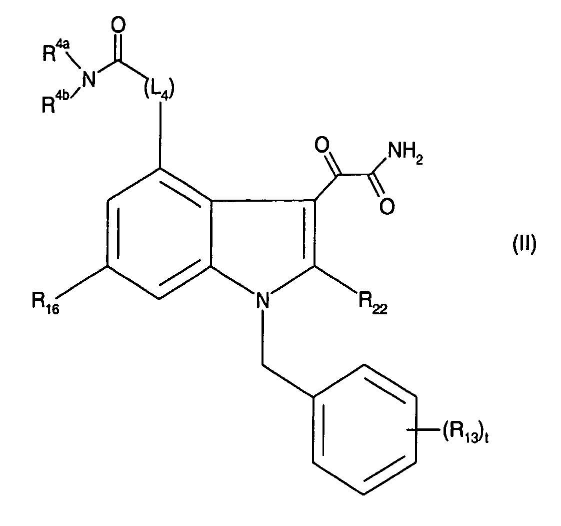 wherein ;
R₂₂ is selected from hydrogen, methyl, ethyl, propyl, isopropyl, cyclopropyl, -F, -CF₃, -Cl, -Br, or -O-CH_{3;}
R^{4a} is hydrogen; and
NR^{4b} is an amino acid residue selected from the group comprising isoleucine, valine, phenylalanine, aspartic acid, leucine, glycine, asparagine, cysteine, glutamine, glutamic acid, histidine, lysine, methionine, serine, threonine, tryptophan, tyrosine *1*-proline, and *d*-proline, and -(L₄)- is a divalent group selected from;
⁅O-CH₂⁆ ,
⁅S-CH₂⁆ , 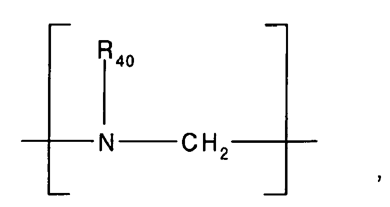 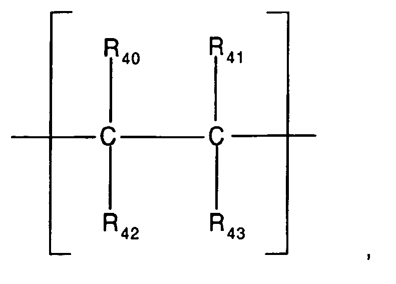
or 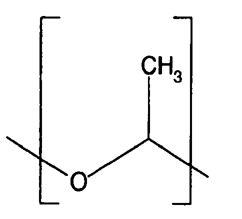 where R₄₀, R₄₁, R₄₂, and R₄₃ are each independently selected from hydrogen or C₁-C₈ alkyl;
R₁₆ is hydrogen; and
R₁₃ is selected from hydrogen and C₁-C₈ alkyl, C₁-C₈ alkoxy, -S-(C₁-C₈ alkyl), C₁-C₈ haloalkyl, C₁-C₈ hydroxyalkyl, phenyl, halophenyl, and halo, and t is an integer from 0 to 5.

6. An indole compound according to claim 1 represented by the formulae (C1), (C2), (C3), (C4), (C5), (C6), (C7), (C8), (C9), (C10) or (C11); 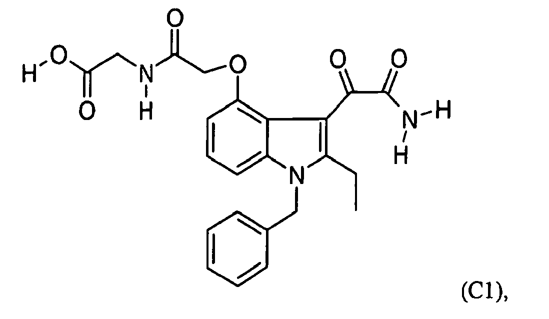 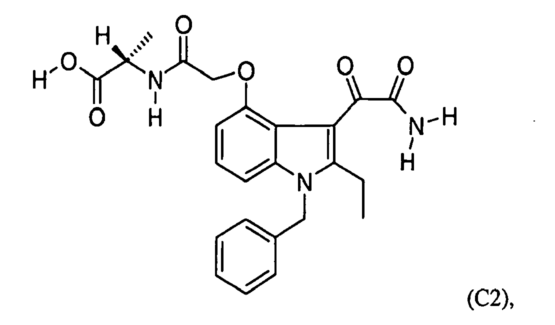 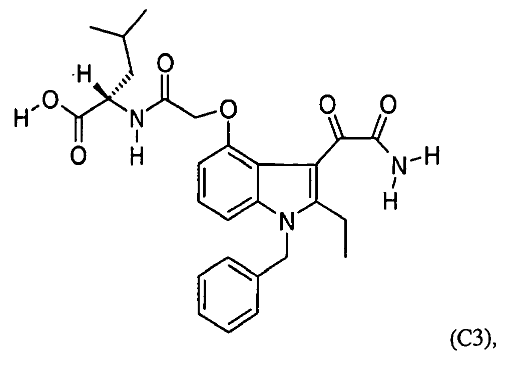 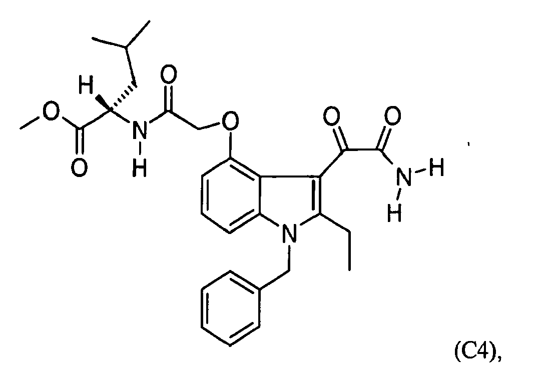 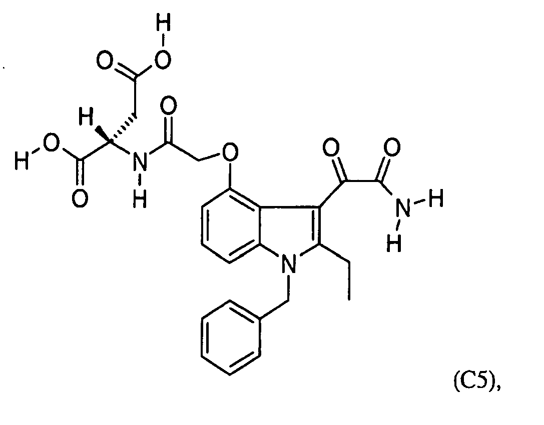 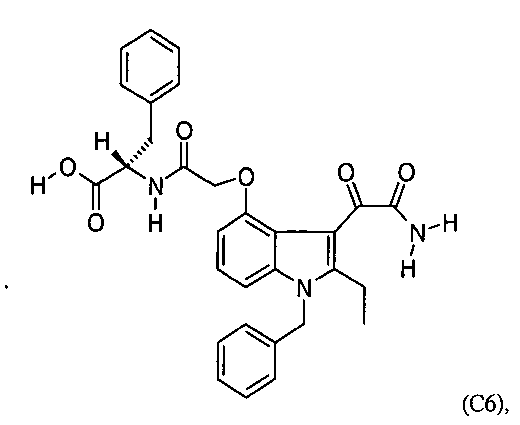 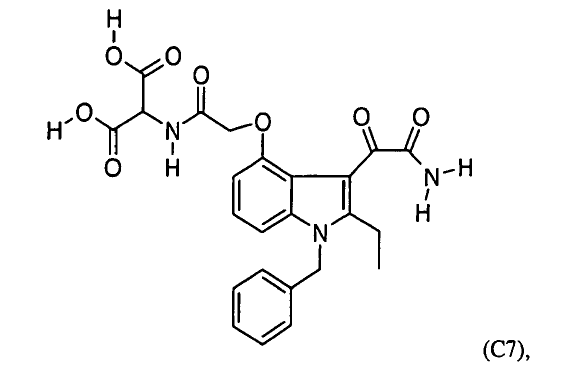 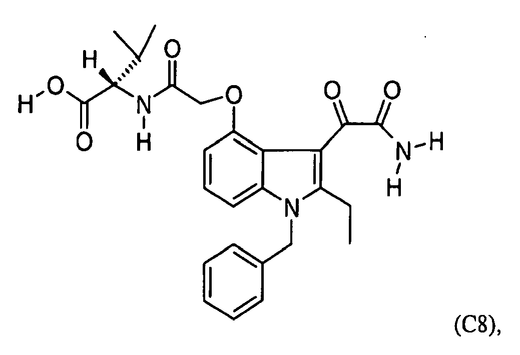 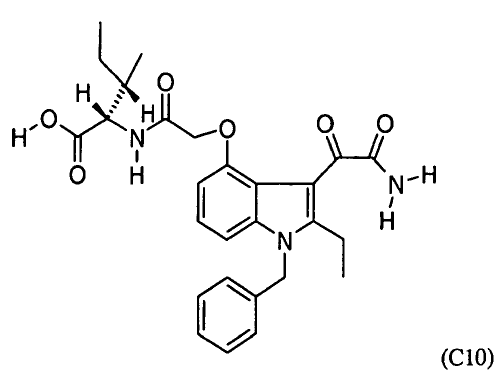 and 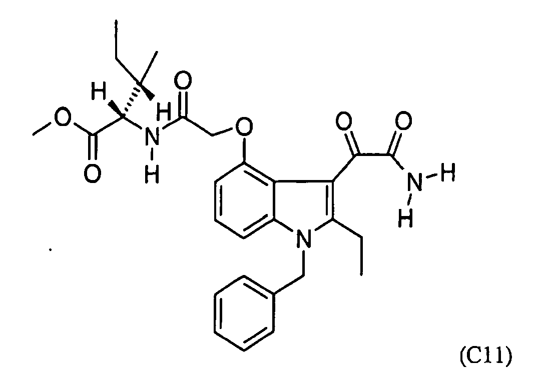 or pharmaceutically acceptable salts or prodrugs selected from the methyl, ethyl, propyl, isopropyl, n-butyl, isobutyl, tert-butyl, morpholinoethyl, and N,N-diethylglycolamido esters thereof.

7. A compound of claim 1 selected from the group consisiting of:
*N*-[2-[[3-(Aminooxoacetyl)-2-ethyl-1-(phenylmethyl)-1H-indol-4-yl]oxy]acetyl]glycine ;
*N*-[2-[[3-(Aminooxoacetyl)-2-ethyl-1-(phenylmethyl)-1H-indol-4-yl]oxy]acetyl]glycine methyl ester;
*N*-[2-[[3-(Aminooxoacetyl)-2-ethyl-1-(phenylmethyl)-1H-indol-4-yl]oxy]acetyl]-L-alanine;
*N*-[2-[[3-(Aminooxoacetyl)-2-ethyl-1-(phenylmethyl)-1H-indol-4-yl]oxy]acetyl]-L-alanine methyl ester;
*N*-[2-[[3-(Aminooxoacetyl)-2-ethyl-1-(phenylmethyl)-1H-indol-4-yl]oxy]acetyl]-L-leucine;
*N*-[2-[[3-(Aminooxoacetyl)-2-ethyl-1-(phenylmethyl)-1H-indol-4-yl]oxy]acetyl]-L-leucine methyl ester;
*N*-[2-[[3-(Aminooxoacetyl)-2-ethyl-1-(phenylmethyl)-1H-indol-4-yl]oxy]acetyl]-L-aspartic acid;
*N*-[2-[[3-(Aminooxoacetyl)-2-ethyl-1-(phenylmethyl)-1H-indol-4-yl]oxy]acetyl]-L-aspartic acid dimethyl ester;
*N*-[2-[[3-(Aminooxoacetyl)-2-ethyl-1-(phenylmethyl)-1H-indol-4-yl]oxy]acetyl]-L-phenylalanine;
*N*-[2-[[3-(Aminooxoacetyl)-2-ethyl-1-(phenylmethyl)-1H-indol-4-yl]oxy]acetyl]-L-phenylalanine methyl ester;
[2-[[3-(Aminooxoacetyl)-2-ethyl-1-(phenylmethyl)-1H-indol-4-yl]oxy]acetamido]malonic acid;
[2-[[3-(Aminooxoacetyl)-2-ethyl-1-(phenylmethyl)-1H-indol-4-yl]oxy]acetamido]malonic acid dimethyl ester
*N*-[2-[[3-(Aminooxoacetyl)-2-ethyl-1-(phenylmethyl)-1H-indol-4-yl]oxy]acetyl]-L-valine;
*N*-[2-[[3-(Aminooxoacetyl)-2-ethyl-1-(phenylmethyl)-1H-indol-4-yl]oxy]acetyl]-L-valine methyl ester;
*N*-[2-[[3-(Aminooxoacetyl)-2-ethyl-1-(phenylmethyl)-1H-indol-4-yl]oxy]acetyl]-L-isoleucine; and
*N*-[2-[[3-(Aminooxoacetyl)-2-ethyl-1-(phenylmethyl)-1H-indol-4-yl]oxy]acetyl]-L-isoleucine methyl ester.

8. A pharmaceutical formulation comprising a indole compound as claimed in claim 1 together with a pharmaceutically acceptable carrier or diluent therefor.

9. Use of an indole compound as claimed in claim 1 for the manufacture of a medicament for inhibiting sPLA₂ mediated release of fatty acid.

10. Use of at least one indole compound as claimed in Claim 1 for the manufacture of a medicament for treating a mammal, including a human, to alleviate the pathological effects of Inflammatory Diseases.

11. A compound of claim 1 or a pharmaceutical formulation containing an effective amount of the compound of claim 1 for use in the treatment of Inflammatory Diseases.

12. A compound of claim 1 or a pharmaceutical formulation containing an effective amount of the compound of claim 1 for use as an inhibitor for inhibiting sPLA₂ mediated release of fatty acid.

## Patentansprüche

1. Indolverbindung, die durch die Formel (I) dargestellt wird, oder ein pharmazeutisch annehmbares Salz, Solvat oder Prodrugderivat, ausgewählt aus den Methyl-, Ethyl-, Propyl-, Isopropyl-, n-Butyl-, Isobutyl-, tert-Butyl-, Morpholinoethyl- und N,N-Diethylglycolamidoestern hiervon 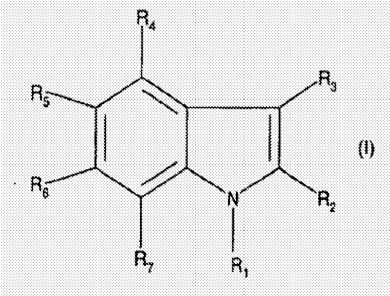 worin
R₁ für die Gruppe -(L₁)-R₁₁ steht, worin -(L₁)- aus einer Gruppe ausgewählt ist, die aus den Formeln (la), (Ib), (Ic), (Id), (Ie) und (If) ausgewählt ist: 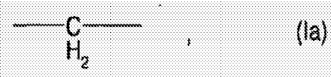
-O- , (Ib)
-S- , (Ic) 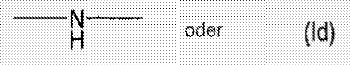 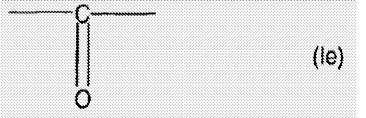
oder 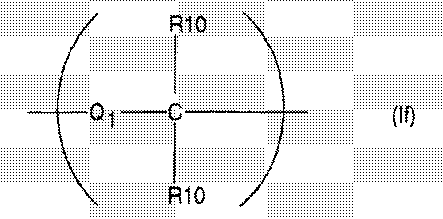 worin Q₁ für eine Bindung oder jede der divalenten Gruppen Ia, Ib, Ic, Id und le steht und R₁₀ unabhängig für -H, C₁-C₈ Alkyl, C₁-C₈ Halogenalkyl oder C₁-C₈ Alkoxy steht und worin R₁₁ für einen substituierten oder unsubstituierten carbocyclischen Rest steht, der aus der Gruppe ausgewählt ist, die besteht aus C₅-C₁₄ Cycloalkyl, C₅-C₁₄ Cycloalkenyl, Phenyl, Spiro[5.5]undecanyl, Naphthyl, Norbornanyl, Bicycloheptadienyl, Toluyl, Xylenyl, Indenyl, Stilbenyl, Terphenylyl, Diphenylethylenyl, Phenylcyclohexenyl, Acenaphthylenyl und Anthracenyl, Biphenyl, Bibenzylyl und verwandten Bibenzylylhomologen, die durch die Formel (a) dargestellt werden 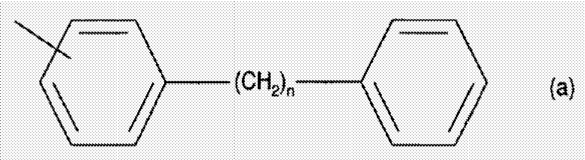 worin n für eine Zahl von 1 bis 8 steht,
R₂ für Wasserstoff, C₁-C₄ Alkyl, C₂-C₄ Alkenyl, -O-(C₁-C₃ Alkyl), -S-(C₁-C₃ Alkyl), C₃-C₄ Cycloalkyl, -CF₃, Halogen, -NO₂, -CN oder -SO₃ steht,
R₃ für -(L₃)-Z steht, worin -(L₃)- für eine Bindung steht und Z aus der Gruppe ausgewählt ist, die durch die folgenden Formeln dargestellt wird 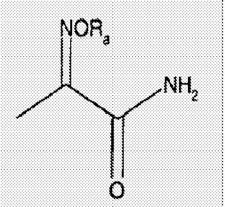 worin Rₐ für Wasserstoff, Methyl, Ethyl, Propyl, Isopropyl, Phenyl oder Benzyl steht 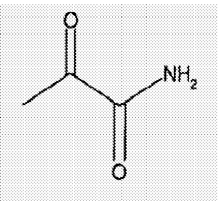 oder 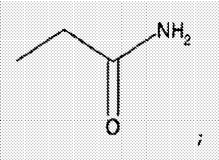 R₄ für die Gruppe -(L_{c})-(Acylaminosäuregruppe) steht, worin -(L_{c})- für eine divalente Gruppe steht, die ausgewählt ist aus
⁅O-CH₂⁆,
⁅S-CH₂⁆, 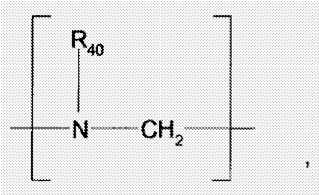 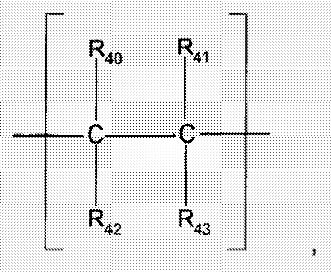
oder 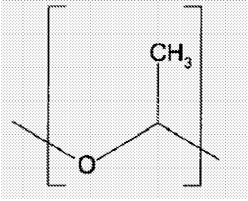 worin R₄₀, R₄₁, R₄₂ und R₄₃ jeweils unabhängig aus Wasserstoff und C₁-C₈ Alkyl ausgewählt sind und worin die (Acylaminosäuregruppe) folgende ist 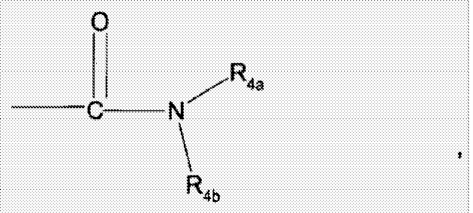 und R^{4a} aus der Gruppe ausgewählt ist, die besteht aus H, C₁-C₆ Alkyl, C₁-C₆ Alkoxy und Aryl und worin NR^{4b} für einen Aminosäurerest steht, der aus der Gruppe ausgewählt ist, die besteht aus Isoleucin, Valin, Phenylalanin, Asparaginsäure, Leucin, Glycin, Asparagin, Cystein, Glutamin, Glutaminsäure, Histidin, Lysin, Methionin, Serin, Threonin, Tryptophan, Tyrosin , L-Prolin und D-Prolin,
R₅ für Wasserstoff steht, und
R₆ und R₇ für Wasserstoff stehen, mit der Maßgabe, dass die folgende Verbindung 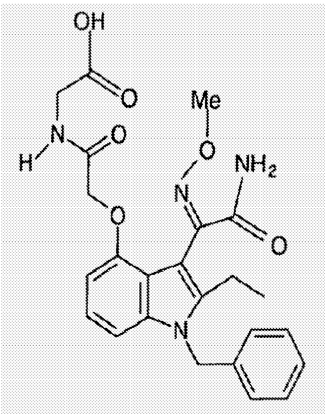 ausgeschlossen ist.

2. Verbindung nach Anspruch 1, worin für R₃, Z die durch die folgende Formel dargestellte Gruppe ist 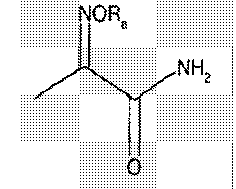 und Rₐ für Wasserstoff steht.

3. Verbindung nach Anspruch 1, worin die Verbindungsgruppe -(L₁)- von R₁ für -(CH₂)- oder -(CH₂-CH₂)-steht.

4. Verbindung nach Anspruch 1, worin für R₁ die kombinierte Gruppe -(L₁)-R₁₁ aus den folgenden Gruppen ausgewählt ist 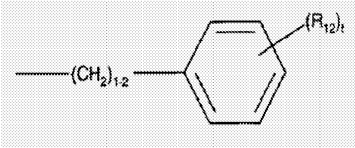 oder 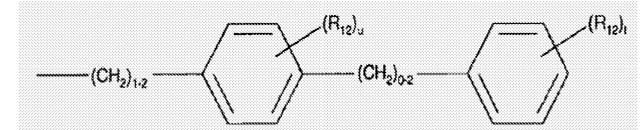 worin R₁₂ für einen Rest steht, der unabhängig aus Halogen, C₁-C₁₀ Alkyl, C₁-C₁₀ Alkoxy, -S-(C₁-C₁₀ Alkyl), C₁-C₁₀ Halogenalkyl und C₁-C₁₀ Hydroxyalkyl ausgewählt ist und t für eine Zahl von 0 bis 5 steht und u für eine Zahl von 0 bis 4 steht.

5. Indolverbindung, die durch die Formel (II) dargestellt wird oder ein pharmazeutisch annehmbares Salz, Solvat oder Prodrug, ausgewählt aus den Methyl-, Ethyl-, Propyl-, Isopropyl-, n-Butyl-, Isobutyl-, tert-Butyl-, Morpholinoethyl- und N,N-Diethylglycolamidoestern hiervon 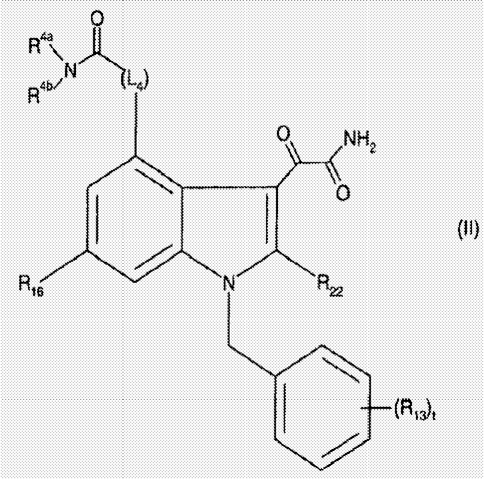 worin R₂₂ ausgewählt ist aus Wasserstoff, Methyl, Ethyl, Propyl, Isopropyl, Cyclopropyl, -F, -CF₃, -Cl, -Br oder -O-CH₃,
R^{4a} für Wasserstoff steht und
NR^{4b} für einen Aminosäurerest steht, der aus der Gruppe ausgewählt ist, die besteht aus Isoleucin, Valin, Phenylalanin, Asparaginsäure, Leucin, Glycin, Asparagin, Cystein, Glutamin, Glutaminsäure, Histidin, Lysin, Methionin, Serin, Threonin, Tryptophan, Tyrosin, L-Prolin und D-Prolin, und -(L₄)- für eine divalente Gruppe steht, die ausgewählt ist aus
⁅O-CH₂⁆,
⁅S-CH₂⁆, 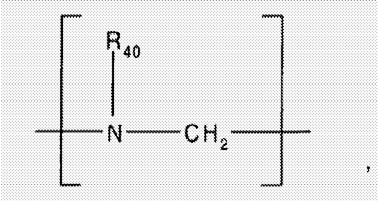 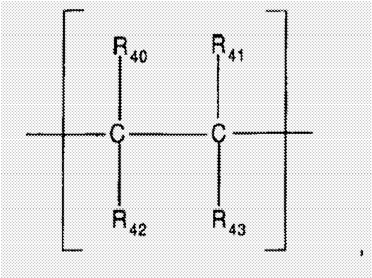
oder 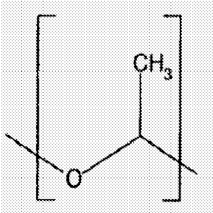 worin R₄₀, R₄₁, R₄₂ und R₄₃ jeweils unabhängig aus Wasserstoff oder C₁-C₈ Alkyl ausgewählt sind,
R₁₆ für Wasserstoff steht und
R₁₃ ausgewählt ist aus Wasserstoff und C₁-C₈ Alkyl, C₁-C₈ Alkoxy, -S-(C₁-C₈ Alkyl), C₁-C₈ Halogenalkyl, C₁-C₈ Hydroxyalkyl, Phenyl, Halogenphenyl und Halogen und t für eine ganze Zahl von 0 bis 5 steht.

6. Indolverbindung nach Anspruch 1, die durch die Formeln (C1), (C2), (C3), (C4), (C5), (C6), (C7), (C8), (C9), (C10) oder (C11) dargestellt ist: 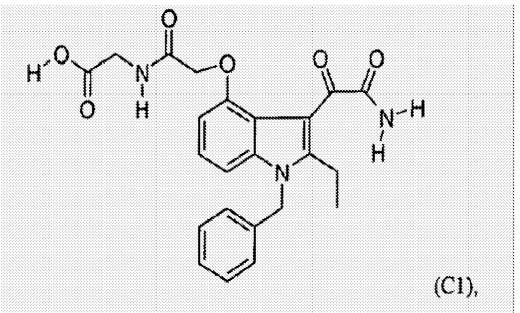 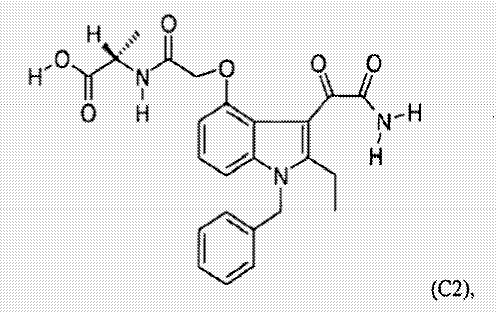 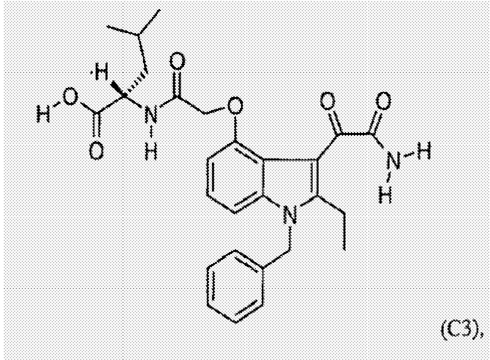 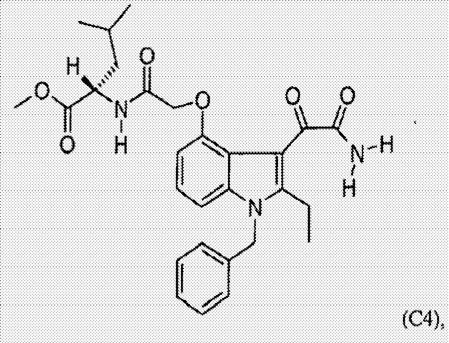 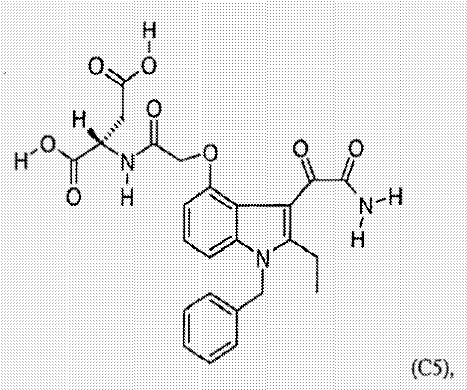 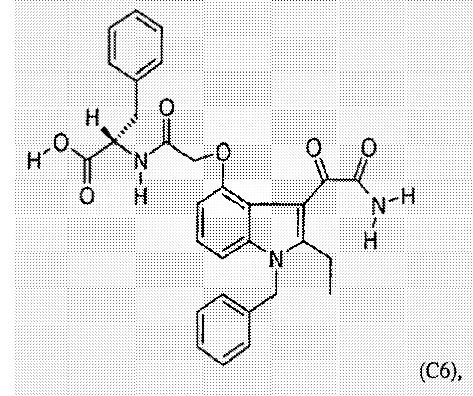 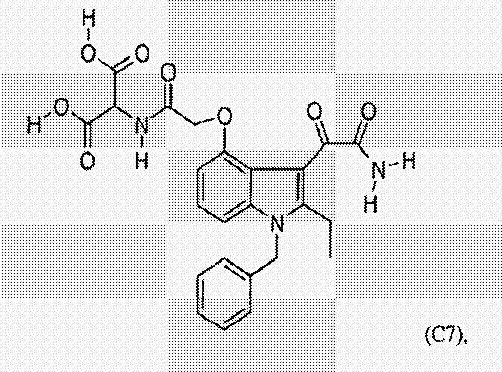 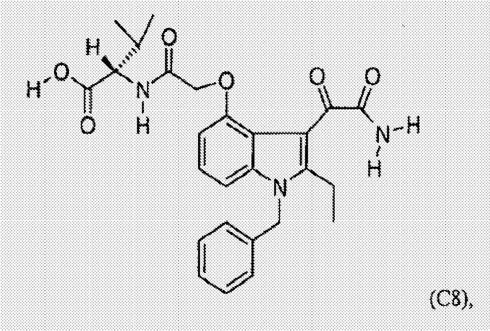 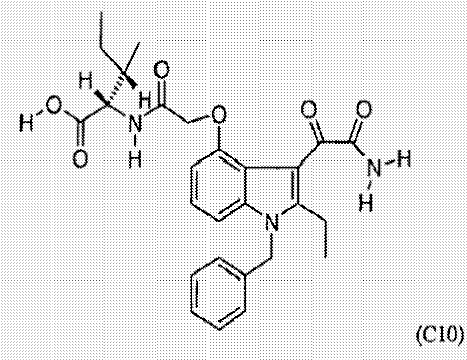 und 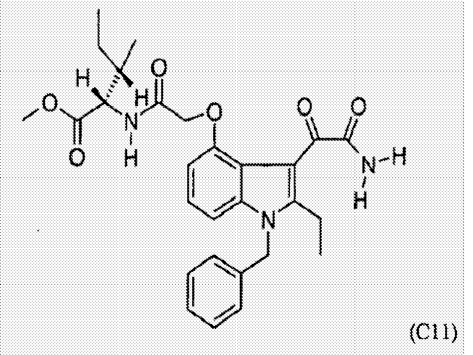 oder pharmazeutisch annehmbare Salze oder Prodrugs hiervon, die ausgewählt sind aus den Methyl-, Ethyl-, Propyl-, Isopropyl-, n-Butyl-, Isobutyl-, tert-Butyl-, Morpholinoethyl und N,N-Diethylglycolamidoestern hiervon.

7. Verbindung nach Anspruch 1, die aus der Gruppe ausgewählt ist, welche besteht aus:
N-[2-[[3-(Aminooxoacetyl)-2-ethyl-1-(phenylmethyl)-1H-indol-4-yl]oxy]acetyl]glycin,
N-[2-[[3-(Aminooxoacetyl)-2-ethyl-1-(phenylmethyl)-1H-indol-4-yl]oxy]acetyl]glycinmeth ylester,
N-[2-[[3-(Aminooxoacetyl)-2-ethyl-1-(phenylmethyl)-1H-indol-4-yl]oxy]acetyl]-L-alanin,
N-[2-[[3-(Aminooxoacetyl)-2-ethyl-1-(phenylmethyl)-1H-indol-4-yl]oxy]acetyl]-L-alaninmethylester,
N-[2-[[3-(Aminooxoacetyl)-2-ethyl-1-(phenylmethyl)-1H-indol-4-yl]oxy]acetyl]-L-leucin,
N-[2-[[3-(Aminooxoacetyl)-2-ethyl-1-(phenylmethyl)-1H-indol-4-yl]oxy]acetyl]-L-leucinmethylester,
N-[2-[[3-(Aminooxoacetyl)-2-ethyl-1-(phenylmethyl)-1H-indol-4-yl]oxy]acetyl]-L-asparaginsäure,
N-[2-[[3-(Aminooxoacetyl)-2-ethyl-1-(phenylmethyl)-1H-indol-4-yl]oxy]acetyl]-L-asparaginsäuredimethylester,
N-[2-[[3-(Aminooxoacetyl)-2-ethyl-1-(phenylmethyl)-1H-indol-4-yl]oxy]acetyl]-L-phenylalanin,
N-[2-[[3-(Aminooxoacetyl)-2-ethyl-1-(phenylmethyl)-1H-indol-4-yl]oxy]acetyl]-L-phenylalaninmethylester,
N-[2-[[3-(Aminooxoacetyl)-2-ethyl-1-(phenylmethyl)-1H-indol-4-yl]oxy]acetamido]-malonsäure,
N-[2-[[3-(Aminooxoacetyl)-2-ethyl-1-(phenylmethyl)-1H-indol-4-yl]oxy]acetamido]-malonsäuredimethylester,
N-[2-[[3-(Aminooxoacetyl)-2-ethyl-1-(phenylmethyl)-1H-indol-4-yl]oxy]acetyl]-L-valin,
N-[2-[[3-(Aminooxoacetyl)-2-ethyl-1-(phenylmethyl)-1H-indol-4-yl]oxy]acetyl]-L-valinmethylester,
N-[2-[[3-(Aminooxoacetyl)-2-ethyl-1-(phenylmethyl)-1H-indol-4-yl]oxy]acetyl]-L-isoleucin, und
N-[2-[[3-(Aminooxoacetyl)-2-ethyl-1-(phenylmethyl)-1H-indol-4-yl]oxy]acetyl]-L-isoleucinmethylester.

8. Pharmazeutische Formulierung, die eine Indolverbindung nach Anspruch 1 zusammen mit einem pharmazeutisch annehmbaren Träger oder Verdünnungsmittel hierfür umfasst.

9. Verwendung einer Indolverbindung nach Anspruch 1 zur Herstellung eines Arzneimittels zur Hemmung der durch sPLA₂ vermittelten Freisetzung von Fettsäuren.

10. Verwendung zumindest einer Indolverbindung nach Anspruch 1 zur Herstellung eines Arzneimittels zur Behandlung eines Säugers, einschließlich eines Menschen, zur Linderung der pathologischen Effekte von Entzündungserkrankungen.

11. Verbindung nach Anspruch 1 oder eine pharmazeutische Formulierung, die eine wirksame Menge der Verbindung nach Anspruch 1 zur Verwendung bei der Behandlung von entzündlichen Erkrankungen enthält.

12. Verbindung nach Anspruch 1 oder eine pharmazeutische Formulierung, die eine wirksame Menge der Verbindung nach Anspruch 1 zur Verwendung als Inhibitor zur Hemmung der sPLA₂ vermittelten Freisetzung von Fettsäure enthält.

## Revendications

1. Composé indole représenté par la formule (I), ou un sel, un solvate ou un pro-médicament dérivé pharmaceutiquement acceptable de celui-ci choisi parmi les esters méthylique, éthylique, propylique, isopropylique, n-butylique, isobutylique, tert-butylique, morpholinoéthylique, et N,N-diéthylglycolamido ; 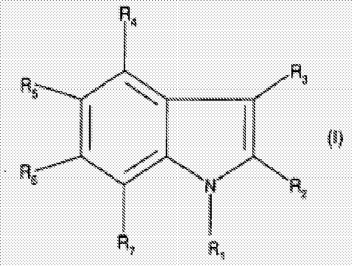 dans laquelle ;
R₁ représente le groupe -(L₁)-R₁₁ ; où -(L₁)- est choisi parmi un groupe représenté par les formules suivantes (Ia), (Ib), (Ic), (Id), (Ie), et (If) : 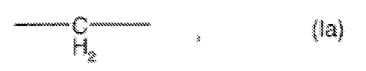
-O- , (Ib)
-S- , (Ic) 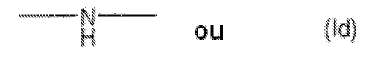 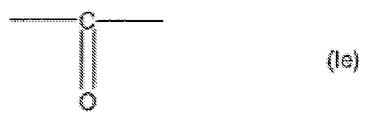
ou 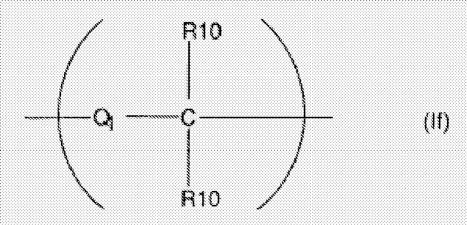 où Q₁ représente une liaison ou n'importe lequel des groupes divalents Ia, Ib, Ic, Id, et Ie, et R₁₀ représente indépendamment H, un groupe alkyle en C₁-C₈ ; un groupe halogénoalkyle en C₁-C₈, ou un groupe alcoxy en C₁-C₈ ; et où R₁₁ représente un radical carbocyclique substitué ou non substitué choisi dans le groupe constitué des groupes cycloalkyle en C₅ à C₁₄, cycloalcényle en C₅ à C₁₄, phényle, spiro[5.5]undécanyle, naphtyle, norbornanyle, bicycloheptadiényle, toluyle, xylényle, indényle, stilbényle, terphénylyle, diphényléthylényle, phénylcyclohexényle, acénaphtylényle, anthracényle, biphényle, bibenzylyle et des homologues bibenzylyle apparentés représentés par la formule (a) : 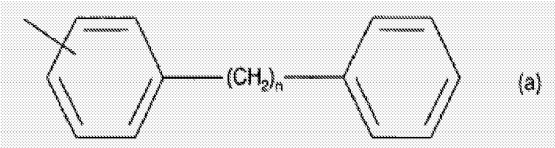 où n est un nombre de 1 à 8 ;
R₂ représente un atome d'hydrogène, un groupe alkyle en C₁-C₄, un groupe alcényle en C₂-C₄, un groupe -O-(alkyle en C₁-C₃), un groupe -S-(alkyle en C₁-C₃), un groupe cycloalkyle en C₃-C₄, -CF₃, un atome d'halogène, -NO₂, -CN, ou -SO₃ ;
R₃ représente -(L₃)-Z, où -(L₃)- représente une liaison et Z est choisi dans un groupe représenté par les formules 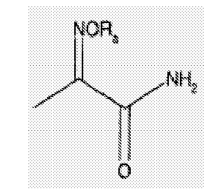 où Rₐ est un atome d'hydrogène, un groupe méthyle, éthyle, propyle, isopropyle, phényle ou benzyle, 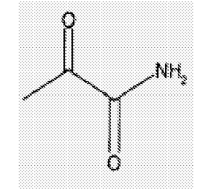 ou 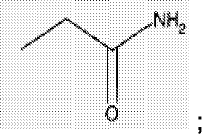 R₄ représente le groupe -(L_{c})-(groupe acide acylaminé); dans lequel -(L_{c})- est un groupe divalent choisi parmi
⁅-O-CH₂-⁆
⁅S-CH₂⁆, 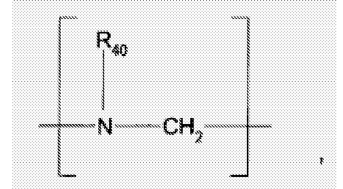 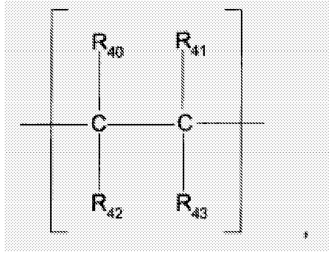
ou 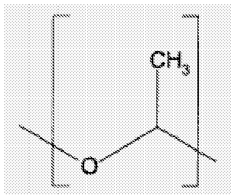 où R₄₀, R₄₁, R₄₂, et R₄₃ sont chacun indépendamment choisis parmi un atome d'hydrogène, un groupe alkyle en C₁-C₈ et dans lequel le (groupe acylaminé) est 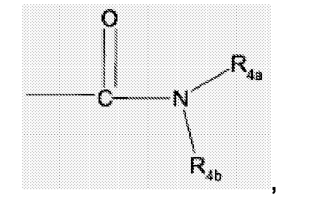 et R^{4a} est choisi dans le groupe constitué de H, un groupe alkyle en C₁-C₆, un groupe alcoxy en C₁-C₆, et un groupe aryle ; et dans lequel NR^{4b} est un résidu acide aminé choisi dans le groupe comprenant l'isoleucine, la valine, la phénylalanine, l'acide aspartique, la leucine, la glycine, l'asparagine, la cystéine, la glutamine, l'acide glutamique, l'histidine, la lysine, la méthionine, la sérine, la thréonine, le tryptophan, la tyrosine, la I-proline, et la d-proline ;
R₅ représente un atome d'hydrogène ; et
R₆ et R₇ représentent un atome d'hydrogène ;
à condition que le composé 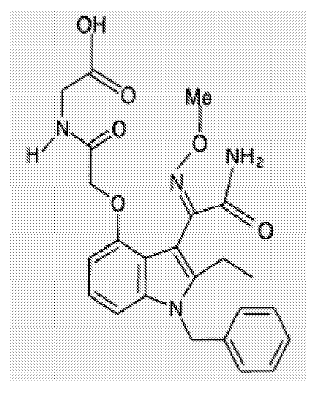 soit exclus.

2. Composé selon la revendication 1, dans lequel pour R₃, Z est le groupe représenté par la formule 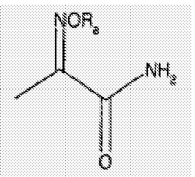 et Rₐ représente un atome d'hydrogène.

3. Composé selon la revendication 1, dans lequel le groupe liant -(L₁)- de R₁ représente -(CH₂)- ou -(CH₂CH₂)-.

4. Composé selon la revendication 1, dans lequel pour R₁, le groupe combiné -(L₁)-R₁₁ est choisi parmi les groupes 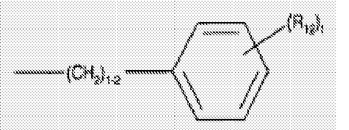 ou 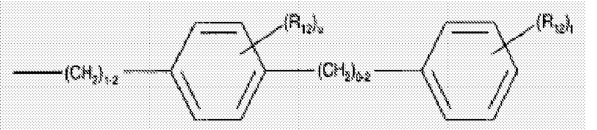 où R₁₂ représente un radical indépendamment choisi parmi un atome d'halogène, un groupe alkyle en C₁-C₁₀, un groupe alcoxy en C₁-C₁₀, un groupe -S-(alkyle en C₁-C₁₀), un groupe halogénoalkyle en C₁-C₁₀, et un groupe hydroxyalkyle en C₁-C₁₀, et t est un nombre entier valant de 0 à 5 et u est un nombre entier valant de 0 à 4.

5. Composé indole représenté par la formule (II), ou un sel, un solvate ou un pro-médicament dérivé pharmaceutiquement acceptable de celui-ci choisi parmi les esters méthylique, éthylique, propylique, isopropylique, n-butylique, isobutylique, tert-butylique, morpholinoéthylique, et N,N-diéthylglycolamido; 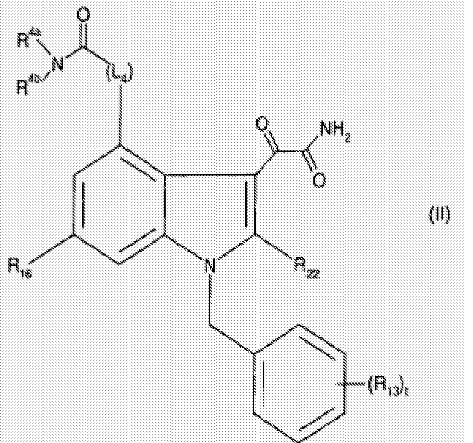 dans laquelle ;
R₂₂ est choisi parmi un atome d'hydrogène, un groupe méthyle, un groupe éthyle, un groupe propyle, un groupe isopropyle, un groupe cyclopropyle, -F, -CF₃, -Cl, -Br, ou -O-CH₃ ;
R^{4a} représente un atome d'hydrogène ; et
NR^{4b} représente un résidu acide aminé choisi dans le groupe comprenant l'isoleucine, la valine, la phénylalanine, l'acide aspartique, la leucine, la glycine, l'asparagine, la cystéine, la glutamine, l'acide glutamique, l'histidine, la lysine, la méthionine, la sérine, la thréonine, le tryptophan, la tyrosine, la I-proline, et la d-proline, et -(L₄)- est un groupe divalent choisi parmi
⁅O-CH₂⁆
⁅S-CH₂⁆ 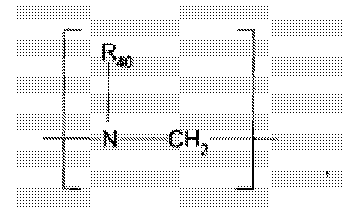 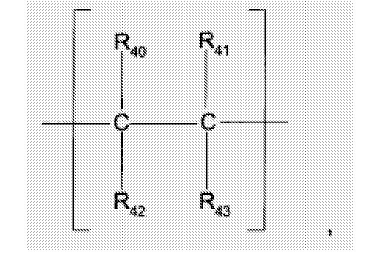
ou 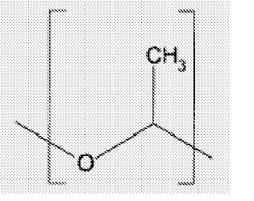 où R₄₀, R₄₁, R₄₂, et R₄₃ sont chacun indépendamment choisis parmi un atome d'hydrogène ou un groupe alkyle en C₁-C₈ ;
R₁₆ représente un atome d'hydrogène ; et
R₁₃ est choisi parmi un atome d'hydrogène et un groupe alkyle en C₁-C₈, un groupe alcoxy en C₁-C₈, un groupe -S-(alkyle en C₁-C₈), un groupe halogénoalkyle en C₁-C₈, un groupe hydroxyalkyle en C₁-C₈, un groupe phényle, un groupe halogénophényle, et un atome d'halogène, et t est un nombre entier valant de 0 à 5.

6. Composé indole selon la revendication 1 représenté par la formule (C1), (C2), (C3), (C4), (C5), (C6), (C7), (C8), (C9), (C10) ou (C11) ; 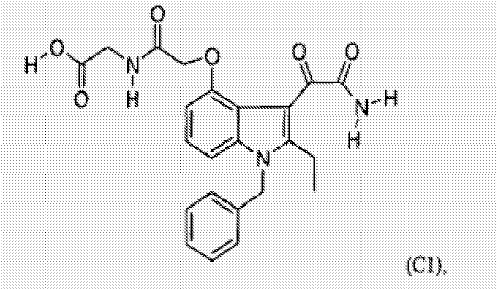 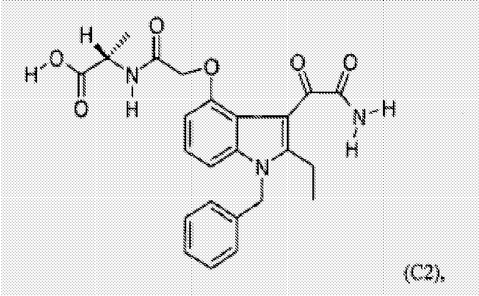 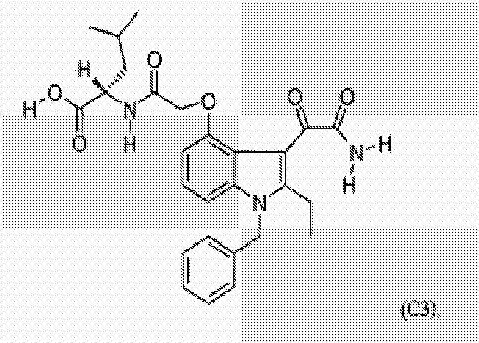 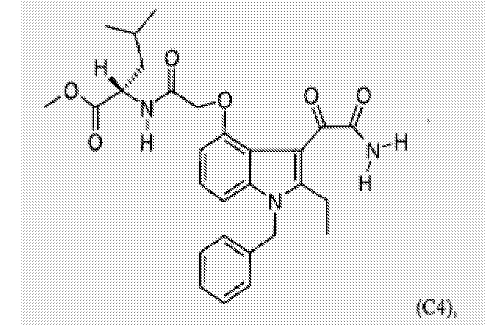 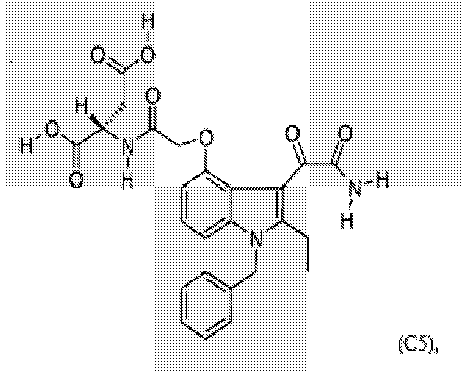 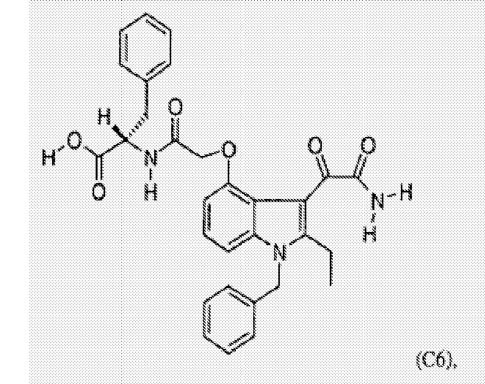 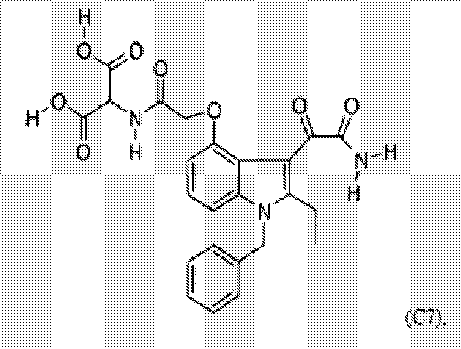 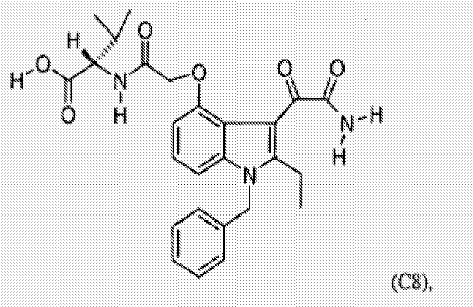 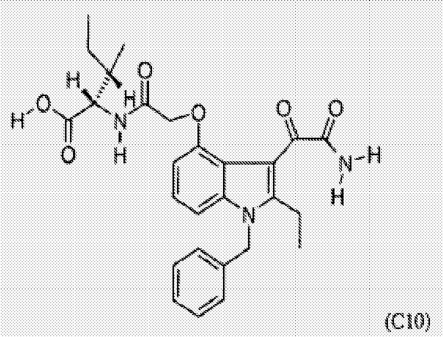 et 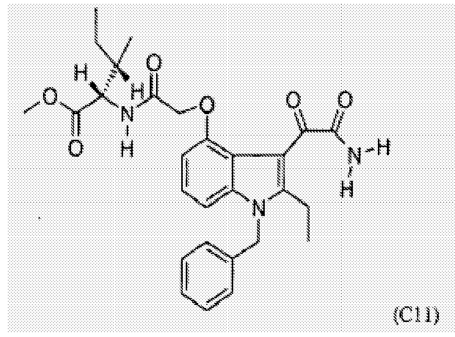 ou des sels ou des pro-médicaments pharmaceutiquement acceptables de ceux-ci choisis parmi les esters méthylique, éthylique, propylique, isopropylique, n-butylique, isobutylique, tert-butylique, morpholinoéthylique, et N,N-diéthylglycolamido.

7. Composé selon la revendication 1 choisi dans le groupe constitué de
la N-[2-[[3-(aminooxoacétyl)-2-éthyl-1-(phénylméthyl)-1H-indol-4-yl]oxy]-acétyl]glycine ;
l'ester méthylique de la N-[2-[[3-(aminooxoacétyl)-2-éthyl-1-(phényl-méthyl)-1 H-indol-4-yl]oxy]acétyl]glycine ;
la N-[2-[[3-(aminooxoacétyl)-2-éthyl-1-(phénylméthyl)-1H-indol-4-yl]oxy]-acétyl]-L-alanine ;
l'ester méthylique de la N-[2-[[3-(aminooxoacétyl)-2-éthyl-1-(phénylméthyl)-1 H-indol-4-yl]oxy]acétyl]-L-alanine ;
la N-[2-[[3-(aminooxoacétyl)-2-éthyl-1-(phénylméthyl)-1H-indol-4-yl]oxy]-acétyl]-L-leucine ;
l'ester méthylique de la N-[2-[[3-(aminooxoacétyl)-2-éthyl-1-(phénylméthyl)-1 H-indol-4-yl]oxy]acétyl]-L-leucine ;
l'acide N-[2-[[3-(aminooxoacétyl)-2-éthyl-1-(phénylméthyl)-1H-indol-4-yl]-oxy]acétyl]-L-aspartique ;
l'ester diméthylique de l'acide N-[2-[[3-(aminooxoacétyl)-2-éthyl-1-(phénylméthyl)-1 H-indol-4-yl]oxy]acétyl]-L-aspartique ;
la N-[2-[[3-(aminooxoacétyl)-2-éthyl-1-(phénylméthyl)-1H-indol-4-yl]oxy]-acétyl]-L-phénylalanine ;
l'ester méthylique de la N-[2-[[3-(aminooxoacétyl)-2-éthyl-1-(phénylméthyl)-1 H-indol-4-yl]oxy]acétyl]-L-phénylalanine ;
l'acide [2-[[3-(aminooxoacétyl)-2-éthyl-1-(phénylméthyl)-1H-indol-4-yl]-oxy]acétamido]malonique ;
l'ester diméthylique de l'acide [2-[[3-(aminooxoacétyl)-2-éthyl-1-(phénylméthyl)-1 H-indol-4-yl]oxy]acétamido]malonique ;
la N-[2-[[3-(aminooxoacétyl)-2-éthyl-1-(phénylméthyl)-1H-indol-4-yl]oxy]-acétyl]-L-valine ;
l'ester méthylique de la N-[2-[[3-(aminooxoacétyl)-2-éthyl-1-(phénylméthyl)-1 H-indol-4-yl]oxy]acétyl]-L-valine ;
la N-[2-[[3-(aminooxoacétyl)-2-éthyl-1-(phénylméthyl)-1H-indol-4-yl]oxy]-acétyl]-L-isoleucine ; et
l'ester méthylique de la N-[2-[[3-(aminooxoacétyl)-2-éthyl-1-(phénylméthyl)-1H-indol-4-yl]oxy]acétyl]-L-isoleucine.

8. Formulation pharmaceutique comprenant un composé indole selon la revendication 1, avec un support ou un diluant pharmaceutiquement acceptable.

9. Utilisation d'un composé indole selon la revendication 1, pour la fabrication d'un médicament destiné à l'inhibition de la libération d'acide gras médiée par sPLA₂.

10. Utilisation d'au moins un composé indole selon la revendication 1, pour la fabrication d'un médicament destiné au traitement d'un mammifère, incluant un être humain, pour soulager les effets pathologiques de maladies inflammatoires.

11. Composé selon la revendication 1 ou formulation pharmaceutique contenant une quantité efficace du composé selon la revendication 1, pour une utilisation dans le traitement de maladies inflammatoires.

12. Composé selon la revendication 1 ou formulation pharmaceutique contenant une quantité efficace du composé selon la revendication 1, pour une utilisation en tant qu'inhibiteur pour inhiber la libération d'acide gras médiée par sPLA₂.
